# EUROPEAN PATENT SPECIFICATION

(11) **EP 4 628 894 B1**
(45) Date of publication and mention of the grant of the patent: **11.11.2026**
(21) Application number: 25157045.3
(22) Date of filing: 11.02.2025
(51) Int. Cl.: G01N 33/536, G01N 33/543, G01N 33/547, G01N 33/566, G01N 33/576, G01N 33/58, G01N 33/68

(54) **AFFINITY PARTICLE, TEST REAGENT, AND DETECTION METHOD FOR DETECTING TARGET IMMUNOGLOBULIN G IN HUMAN SPECIMEN**
AFFINITÄTSPARTIKEL, TESTREAGENS UND NACHWEISVERFAHREN ZUM NACHWEIS VON ZIELIMMUNGLOBULIN G IN MENSCHLICHEN PROBEN
PARTICULE D'AFFINITÉ, RÉACTIF DE TEST ET PROCÉDÉ DE DÉTECTION POUR DÉTECTER UNE IMMUNOGLOBULINE G CIBLE DANS UN SPÉCIMEN HUMAIN

(30) Priority: 15.02.2024 JP 2024021390
(43) Date of publication of application: 08.10.2025
(73) Proprietor: CANON KABUSHIKI KAISHA, Tokyo 146-8501 (JP)
(72) Inventor: SUGIYAMA, Narumi, Tokyo (JP); KAKU, Mai, Tokyo (JP); KATO, Yuki, Tokyo (JP); KAWABE, Yudai, Tokyo (JP)
(74) Representative: WESER & Kollegen Patentanwälte PartmbB

(56) References cited:
- EP-A1- 4 023 682
- JP-A- 2013 148 496
- JP-A- H0 894 619
- US-A1- 2010 216 257
- US-B1- 6 379 886

## Description

### BACKGROUND OF THE INVENTION

### Field of the Invention

The present invention relates to an affinity particle, a test reagent, and a detection method for detecting target immunoglobulin G in a human specimen.

### Description of the Related Art

The test (antibody test) of a specific immunoglobulin present in a human specimen has been widely used in an infectious disease-related test, such as: the antibody test of a hepatitis virus such as a hepatitis C virus (HCV) or a hepatitis B virus (HBV); or the antibody test of a *Treponema pallidum* antigen. Particularly when the test is used as a screening test for a group or an infection recognition test for preventing infection in a medical procedure such as an operation, it is preferred that the test can be simply and rapidly performed.

A simple and rapid immunological test method is, for example, an immunological latex agglutination measurement method (hereinafter referred to as "latex agglutination method"). In the method, when a target substance is a specific antibody (immunoglobulin) present in a human specimen as described above, the target substance (immunoglobulin) in the specimen and a latex particle having carried thereon an antigen having an affinity for the target substance are caused to react with each other. Then, a diagnosis is made by optically detecting an agglutination reaction between the particles through the antigen-antibody reaction as the amount of a change in, for example, scattered light intensity, transmitted light intensity, or absorbance.

For example, the measurement of a HCV-specific immunoglobulin in a human specimen by the latex agglutination method has been performed.

In Japanese Patent Application Laid-Open No. H11-258241, a HCV-specific immunoglobulin is detected with a sensitized particle obtained by: sensitizing a recombinant HCV antigen to a polystyrene latex particle; and then blocking the resultant with bovine serum albumin (BSA). There is a disclosure of a technology in which an improvement in stability of a test drug in long-term storage is achieved by freeze-drying the sensitized particle to suppress a reduction in detection amount due to the peeling or oxidation of the sensitized antigen.

In Japanese Patent Application Laid-Open No. 2013-148496, there is a disclosure of a technology in which in a test reagent using a HCV antigen-sensitized polystyrene latex particle, a specific fatty acid (salt) is used in a measuring system, and hence a nonspecific reaction can be strongly suppressed even when the polystyrene particle is not covered with any blocking agent.

JP H08 94619 A discloses a dried immunological agglutination reagent comprising an immunological agglutination particle carrying an antigen or an antibody and magnetic particles.

In the related art, the range (measurement range) of a measurable HCV-specific immunoglobulin amount has been susceptible to improvement.

### SUMMARY OF THE INVENTION

The present invention has been made in view of such background art and problem. An object of the present invention is to provide a test reagent for a latex agglutination method, the reagent being excellent in sensitivity of the detection of target immunoglobulin G in a human specimen and being capable of sufficiently securing a range (measurement range) in which a detection amount increases with an increase in amount of the target immunoglobulin G. Another object of the present invention is to provide a detection method for detecting target immunoglobulin G in a human specimen.

The present invention relates to an affinity particle for detecting immunoglobulin G in a human specimen by a latex agglutination method, wherein the affinity particle has a volume-average particle diameter of 400 nm or less, wherein the affinity particle includes a latex particle and a protein carried on a surface of the latex particle, wherein the protein contains an antigen having a molecular weight of 10,000 or more, and wherein an amount of the protein carried on the surface of the latex particle is 1.0 µg or more and 20.0 µg or less per 1 mg of the affinity particle.

The present invention also relates to a test reagent including a first reagent liquid having dispersed thereinto the above-mentioned affinity particle.

The present invention also relates to a detection method for detecting immunoglobulin G in a human specimen by a latex agglutination method through use of a test reagent including a first reagent liquid having dispersed thereinto an affinity particle and a second reagent liquid that is a buffer solution, the detection method including: a first step of obtaining a mixed liquid containing the human specimen and the second reagent liquid; a second step of mixing the mixed liquid and the first reagent liquid to cause the mixed liquid and the first reagent liquid to react with each other, to thereby provide a reaction liquid; and a third step of measuring a concentration of the immunoglobulin G in the reaction liquid, wherein the affinity particle has a volume-average particle diameter of 400 nm or less, wherein the affinity particle includes a latex particle and a protein carried on a surface of the latex particle, wherein the protein contains an antigen having a molecular weight of 10,000 or more, and wherein an amount of the protein carried on the surface of the latex particle is 1.0 µg or more and 20.0 µg or less per 1 mg of the affinity particle.

Further features of the present invention will become apparent from the following description of exemplary embodiments with reference to the attached drawings.

### BRIEF DESCRIPTION OF THE DRAWINGS

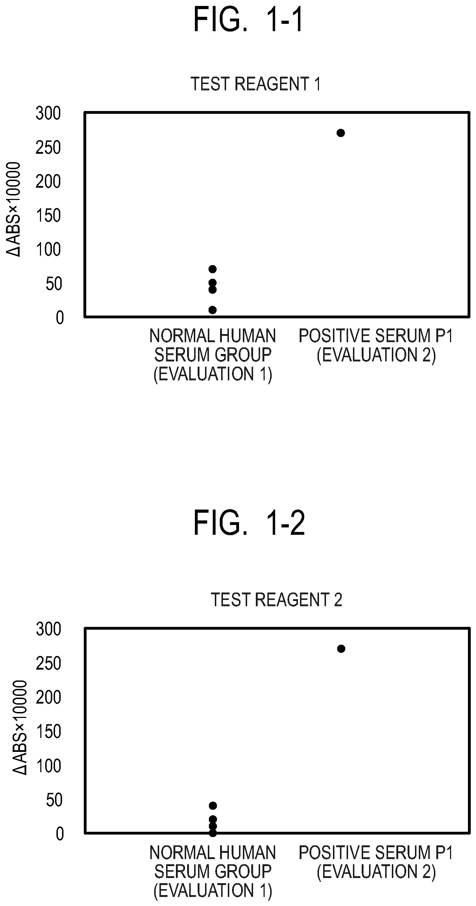

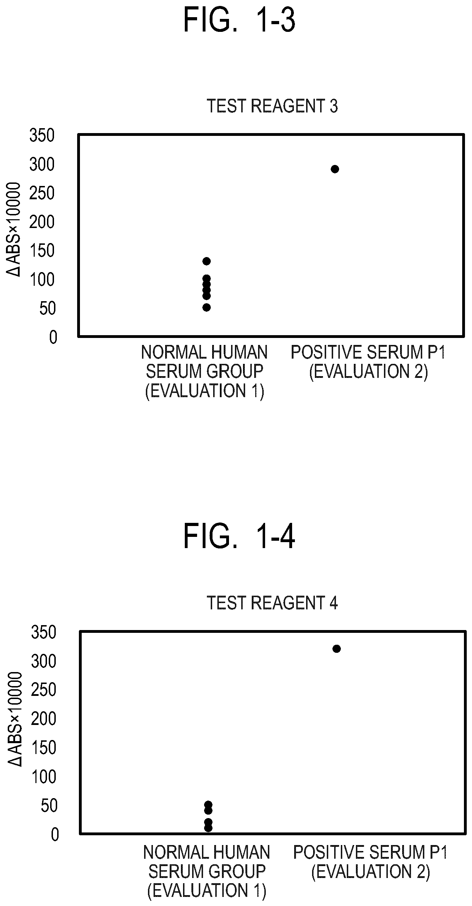

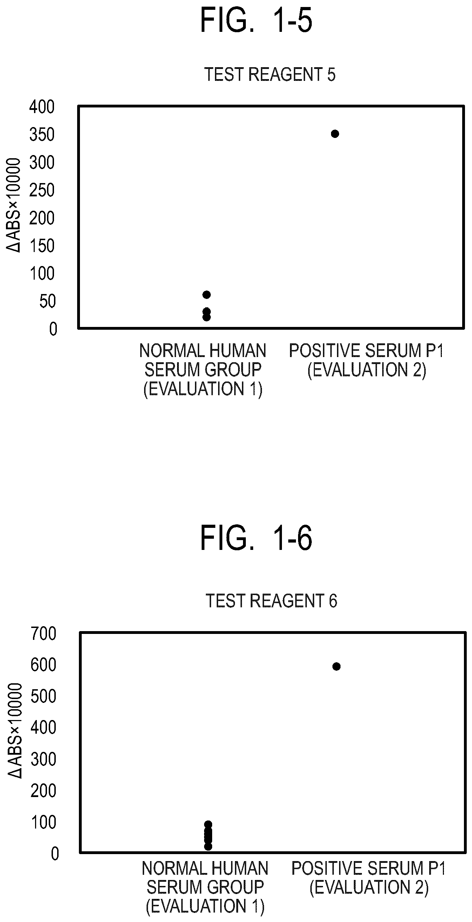

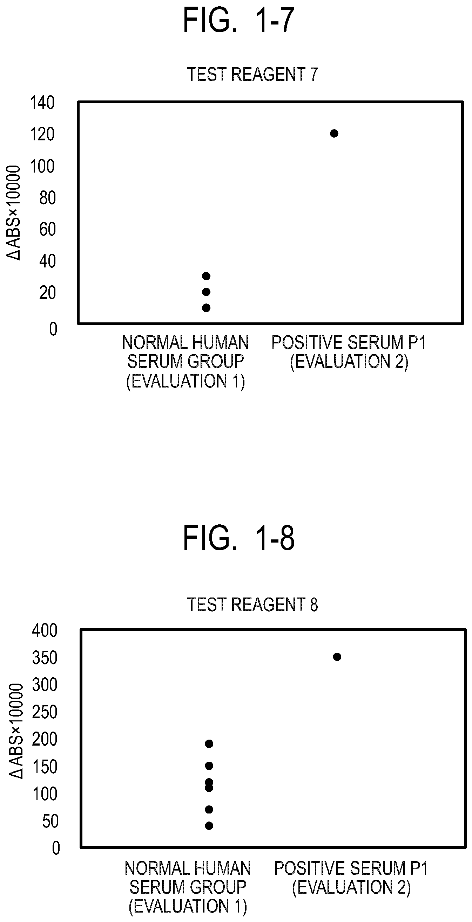

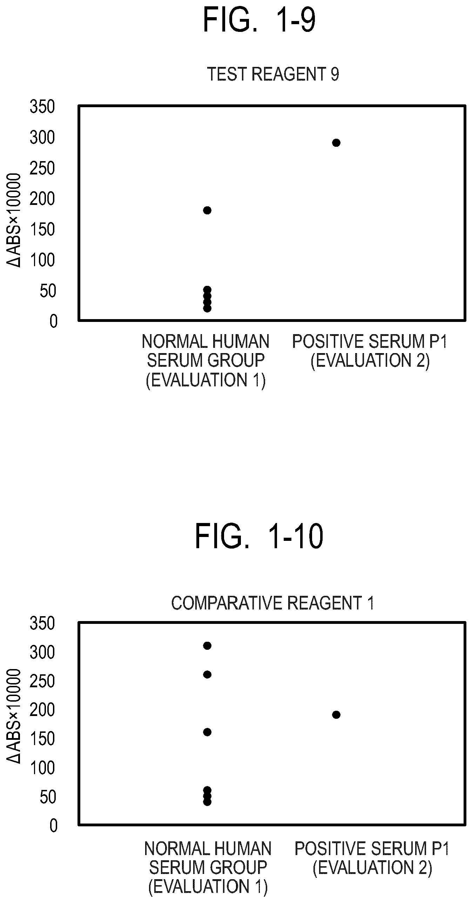

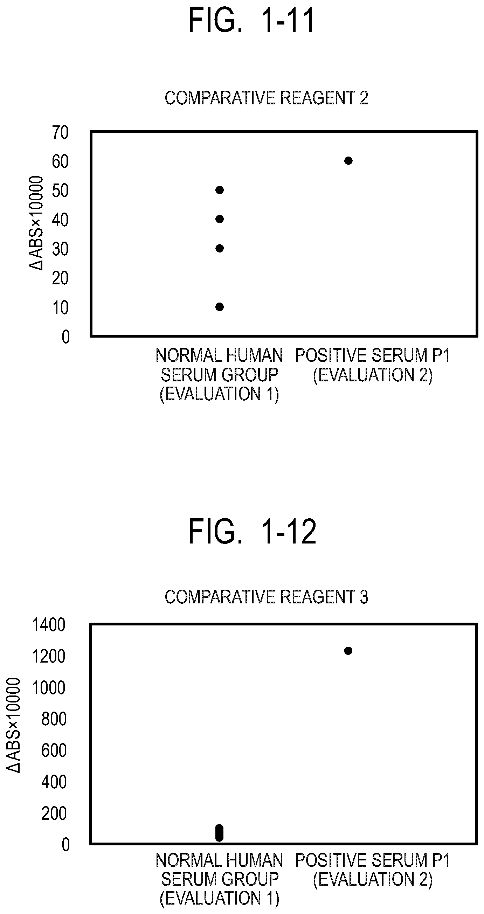

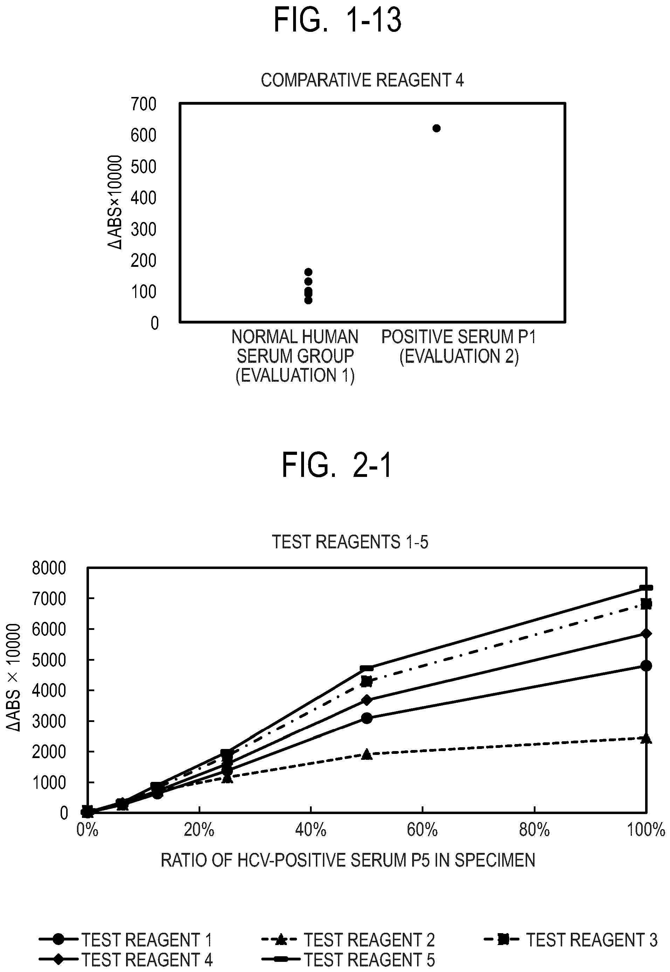

FIG. 1-1 is a graph showing the results of the measurement of a normal human serum group consisting of six kinds of serums (Evaluation 1) and a positive serum P1 (positive low value) (Evaluation 2) with a test reagent 1 according to Example 1 of the present invention.
FIG. 1-2 is a graph showing the results of the measurement of the normal human serum group consisting of six kinds of serums (Evaluation 1) and the positive serum P1 (positive low value) (Evaluation 2) with a test reagent 2 according to Example 2 of the present invention.
FIG. 1-3 is a graph showing the results of the measurement of the normal human serum group consisting of six kinds of serums (Evaluation 1) and the positive serum P1 (positive low value) (Evaluation 2) with a test reagent 3 according to Example 3 of the present invention.
FIG. 1-4 is a graph showing the results of the measurement of the normal human serum group consisting of six kinds of serums (Evaluation 1) and the positive serum P1 (positive low value) (Evaluation 2) with a test reagent 4 according to Example 4 of the present invention.
FIG. 1-5 is a graph showing the results of the measurement of the normal human serum group consisting of six kinds of serums (Evaluation 1) and the positive serum P1 (positive low value) (Evaluation 2) with a test reagent 5 according to Example 5 of the present invention.
FIG. 1-6 is a graph showing the results of the measurement of the normal human serum group consisting of six kinds of serums (Evaluation 1) and the positive serum P1 (positive low value) (Evaluation 2) with a test reagent 6 according to Example 6 of the present invention.
FIG. 1-7 is a graph showing the results of the measurement of the normal human serum group consisting of six kinds of serums (Evaluation 1) and the positive serum P1 (positive low value) (Evaluation 2) with a test reagent 7 according to Example 7 of the present invention.
FIG. 1-8 is a graph showing the results of the measurement of the normal human serum group consisting of six kinds of serums (Evaluation 1) and the positive serum P1 (positive low value) (Evaluation 2) with a test reagent 8 according to Example 8 of the present invention.
FIG. 1-9 is a graph showing the results of the measurement of the normal human serum group consisting of six kinds of serums (Evaluation 1) and the positive serum P1 (positive low value) (Evaluation 2) with a test reagent 9 according to Example 9 of the present invention.
FIG. 1-10 is a graph showing the results of the measurement of the normal human serum group consisting of six kinds of serums (Evaluation 1) and the positive serum P1 (positive low value) (Evaluation 2) with a comparative reagent 1 in the related art.
FIG. 1-11 is a graph showing the results of the measurement of the normal human serum group consisting of six kinds of serums (Evaluation 1) and the positive serum P1 (positive low value) (Evaluation 2) with a comparative reagent 2 in the related art.
FIG. 1-12 is a graph showing the results of the measurement of the normal human serum group consisting of six kinds of serums (Evaluation 1) and the positive serum P1 (positive low value) (Evaluation 2) with a comparative reagent 3 in the related art.
FIG. 1-13 is a graph showing the results of the measurement of the normal human serum group consisting of six kinds of serums (Evaluation 1) and the positive serum P1 (positive low value) (Evaluation 2) with a comparative reagent 4 in the related art.
FIG. 2-1 is a graph showing the results of the measurement of a serum N (negative control) (Evaluation 1) and positive serums P1 to P5 (Evaluation 2) with the test reagents 1 to 5 according to Examples 1 to 5 of the present invention.
FIG. 2-2 is a graph showing the results of the measurement of the serum N (negative control) (Evaluation 1) and the positive serums P1 to P5 (Evaluation 2) with the test reagents 6 to 9 according to Examples 6 to 9 of the present invention.

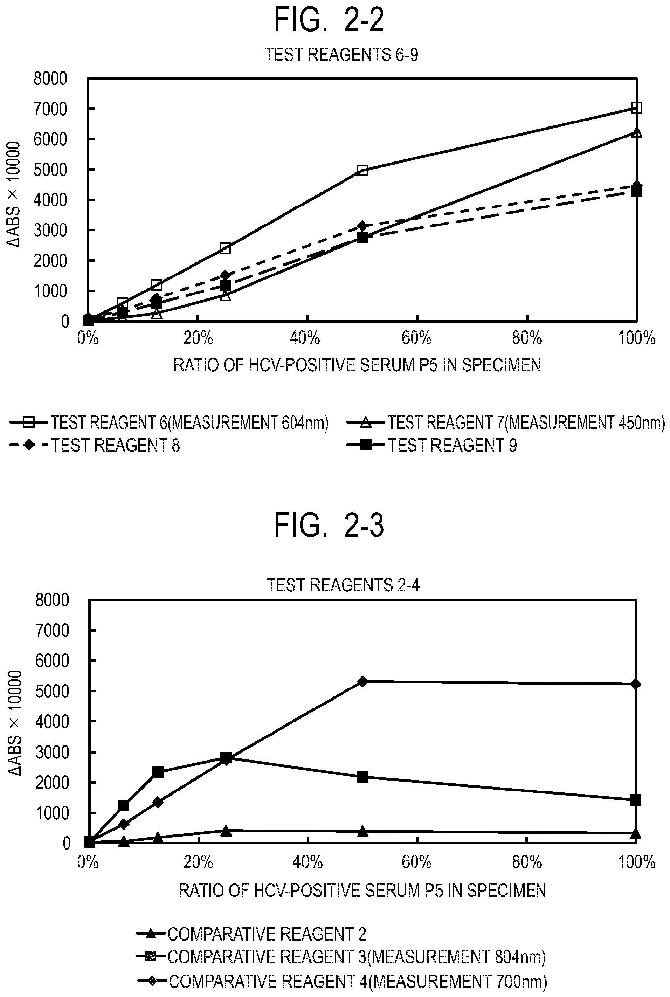

FIG. 2-3 is a graph showing the results of the measurement of the serum N (negative control) (Evaluation 1) and the positive serums P1 to P5 (Evaluation 2) with the comparative reagents 2 to 4 in the related art.

### DESCRIPTION OF THE EMBODIMENTS

Embodiments of the present invention are described in detail below, but the technical scope of the present invention is not limited to these embodiments. In addition, the description "(meth)acrylate" as used herein refers to a methacrylate and/or an acrylate, and for example, the description "glycidyl (meth)acrylate" refers to glycidyl methacrylate and/or glycidyl acrylate.

### <Problems to be solved by Affinity Particle according to this Embodiment>

Problems to be solved by an affinity particle according to this embodiment are described. Herein, in each of Japanese Patent Application Laid-Open No. H11-258241 and Japanese Patent Application Laid-Open No. 2013-148496 described above, no reference is made to the range (measurement range) of a measurable HCV-specific immunoglobulin amount.

The judgment of whether a subject is negative or positive in a HCV antibody test is an extremely effective method of rapidly recognizing that the subject is negative. However, when the subject is judged to be positive, his or her past history is included in the judgment result, and hence current infection cannot be finalized with the result alone. To definitively diagnose the infection, whether or not the subject is infected needs to be judged together with any other method such as a nucleic acid test to be subsequently performed. Meanwhile, when antibody "abundances" in positive specimens can be further classified into groups in the course of the antibody test, the presence or absence of the current infection can be predicted at a certain level, and hence a contribution can be made to an improvement in efficiency of the definitive diagnosis.

Accordingly, a test reagent that can classify the abundances of HCV antibodies in specimens into groups is preferred as a HCV antibody test drug. However, particularly in the case of a test reagent whose measurement principle is a latex agglutination method, it is difficult to achieve both of the securement of a measurement range and an improvement in detection sensitivity, and hence the achievement has been a problem.

When a latex particle having a large particle diameter is used for improving the detection sensitivity, an absorbance change amount increases even in the case where the amount of an antibody in a specimen is small, that is, the number of agglutination reactions is small. Accordingly, an improvement in detection sensitivity can be expected. However, the absorbance of the original particle (before the agglutination) is large, and the specific area of the particle on which an antigen is carried reduces. Accordingly, a range (measurement range) in which the absorbance change amount increases with an increase in antibody amount may narrow. In each of Japanese Patent Application Laid-Open No. H11-258241 and Japanese Patent Application Laid-Open No. 2013-148496 described above, there is a disclosure of a test drug using polystyrene latex having a size of 0.48 µm. The inventors of the present invention have made an investigation to find that when a particle having such size is used, excellent detection sensitivity is obtained, but it is difficult to sufficiently secure a range (measurement range) in which a detection amount increases with an increase in antibody amount.

### <Amount of Protein carried on Surface of Latex Particle>

An affinity particle according to one embodiment of the present invention is an affinity particle for detecting immunoglobulin G in a human specimen by a latex agglutination method, wherein the affinity particle has a volume-average particle diameter of 400 nm or less, wherein the affinity particle includes a latex particle and a protein carried on a surface of the latex particle, wherein the protein contains an antigen having a molecular weight of 10,000 or more for detecting the immunoglobulin G, and wherein an amount of the protein carried on the surface of the latex particle is 1.0 µg or more and 20.0 µg or less per 1 mg of the affinity particle.

The protein carried on the surface of the latex particle contains the antigen for detecting the immunoglobulin G.

The immunoglobulin G serving as a target substance in the present invention is a substance having two antigen-recognizing sites in a molecule thereof. At the time of the measurement of the substance by the latex agglutination method, to improve detection sensitivity, it is important that the two antigen-recognizing sites of a molecule of the immunoglobulin G each react with a separate particle to efficiently cause particle agglutination through the immunoglobulin G. Accordingly, the antigen for detecting the immunoglobulin G, the antigen being carried on the surface of the latex particle, is preferably present in such a state that the molecules of the antigen are sufficiently distant from each other.

When the molecules of the antigen are densely present on the particle, it is assumed that the bonding of the immunoglobulin G serving as the target substance is completed in one particle. The reaction does not lead to the occurrence of particle agglutination, and hence the detection amount of the target substance reduces. Accordingly, excellent detection sensitivity is hardly obtained. In addition, when the molecules of the antigen are present under an agglutinated state on the particle, the nonspecific adsorption of a protein except the target substance is liable to occur. The nonspecific adsorption causes undesired particle agglutination, and hence the detection values of a specimen group free of the target substance in which the detection amounts should originally be 0 are increased, and a variation between the detection amounts is made larger. In that case, it becomes difficult to detect a difference between a specimen free of the target substance and a specimen in which the amount of the target substance is small. After all, excellent detection sensitivity is hardly obtained.

In the present invention, a protein except the above-mentioned antigen (e.g., albumin to be used for particle-blocking treatment) may be incorporated into a reagent liquid having dispersed thereinto the affinity particle, but its amount is preferably as small as possible. Specifically, the ratio of the antigen in the protein carried on the surface of the latex particle is preferably 70 mass% or more because the reactivity of the antigen with the immunoglobulin G serving as the target substance is improved.

The amount of the protein carried on the surface of the latex particle may be determined as the amount of the protein in terms of bovine serum albumin (BSA) by a measurement method to be described later.

It is preferred that the affinity particle for detecting the immunoglobulin G in the human specimen by the latex agglutination method according to the present invention have a volume-average particle diameter of 400 nm or less, and include the latex particle and the antigen carried on the surface of the latex particle, the antigen having a molecular weight of 10,000 or more, and the amount of the antigen carried on the surface of the latex particle be 1.0 µg or more and 20.0 µg or less per 1 mg of the affinity particle. Thus, efficient particle agglutination through the immunoglobulin G serving as the target substance can be expected, and hence excellent detection sensitivity can be obtained.

For example, when the reagent liquid having dispersed thereinto the affinity particle is free of a protein except the antigen for detecting the immunoglobulin G, the amount of the antigen carried on the surface of the latex particle is 1.0 µg or more and 20.0 µg or less per 1 mg of the affinity particle. That is, the ratio of the above-mentioned antigen in the protein carried on the surface of the latex particle is more preferably 100 mass%.

### <Volume-average Particle Diameter of Affinity Particle>

The volume-average particle diameter of the affinity particle according to the present invention having carried thereon the antigen is 400 nm or less. When the volume-average particle diameter is 400 nm or less, a sufficient particle specific surface area can be held. Accordingly, as described above, coupled with the optimization of an antigen-carrying amount, an appropriate interval between the molecules of the antigen on the particle can be kept. Thus, nonspecific adsorption in the latex agglutination method is alleviated, and hence increases in detection amounts of the specimen group free of the target substance and the variation between the detection amounts can be alleviated. Accordingly, the difference between the specimen group free of the target substance and the specimen in which the amount of the target substance is small can be detected with high sensitivity. Further, the sufficient particle specific surface area is extremely suitable because a range (measurement range of the amount of the immunoglobulin G) in which a detection amount increases with an increase in amount of the target immunoglobulin G in the specimen widens. Meanwhile, when the volume-average particle diameter is more than 400 nm, the absorbance of the particle may reduce in a region where the concentration of the target immunoglobulin G is high.

In addition, the volume-average particle diameter is preferably 100 nm or more. When a particle aggregate after a reaction between the target substance and the antigen is small, a difference between the absorbances of the particles before and after their agglutination is also small, and hence sensitivity in a region where the concentration of the target substance is low is hardly improved in some cases. The volume-average particle diameter of the affinity particle according to the present invention may be set to 200 nm or more.

The volume-average particle diameter of the affinity particle dispersed into the reagent liquid may be measured by a measurement method to be described later.

### <Latex Particle before Carriage of Antigen>

The latex particle before the carriage of the antigen in the present invention is a high-molecular weight particle that can carry the antigen to the target substance, and any one of latex particles to be used in typical latex agglutination method reagents may be used. The latex particle before the carriage of the antigen may be synthesized by polymerizing a polymerizable monomer. For example, a latex particle having a core-shell structure synthesized as follows may be used as the latex particle before the carriage of the antigen: the polymerizable monomer is polymerized to synthesize a core particle; and another polymerizable monomer (e.g., glycidyl (meth)acrylate) is further caused to react with the core particle to form a shell. That is, the latex particle has a skeleton structure formed of a structural unit derived from the polymerizable monomers. Examples of the latex particle before the carriage of the antigen include a polystyrene particle, a polystyrene particle having a siloxane, and a polystyrene particle having polyglycidyl (meth)acrylate. Those particles are each a particle that may be suitably used in the affinity particle or a latex agglutination method test reagent in the present invention because the particles each have such advantages as described below: a nanosized particle is relatively easily obtained; and the surface of the particle can be chemically modified in accordance with purposes.

Of those, a latex particle having a structure derived from polyglycidyl (meth)acrylate is preferred because the surface of the particle can be chemically subjected to hydrophilic treatment by causing a specific compound to react with the particle through utilization of a glycidyl group. In particular, the antigen is preferably carried by using, as the latex particle before the carriage of the antigen, a particle containing a polymer having a glycidyl (meth)acrylate-derived structural unit represented by the following formula (1).

In addition, the latex particle preferably further has, as the skeleton structure formed of the structural unit derived from the polymerizable monomers, a structural unit derived from, for example, a styrene, 1-vinylnaphthalene, or 2-vinylnaphthalene because the sensitivity is improved from the viewpoint of a particle refractive index.

The latex particle preferably further has a crosslinked structure as a skeleton structure in addition to those described above. The crosslinked structure is obtained by polymerizing a crosslinkable monomer having two or more radically polymerizable unsaturated bonds in a molecule thereof. Examples of the crosslinkable monomer include divinylbenzene, diethynylnaphthalene, diallyl phthalate, allyl acrylate, allyl methacrylate, a polyfunctional (meth)acrylate, and a conjugated diolefin. The presence of the crosslinked structure makes the particle physically strong to eliminate concern that the particle is broken or chipped by being repeatedly subjected to a centrifugal operation at the time of its purification.

The affinity particle according to the present invention may be an affinity particle, wherein the latex particle has a skeleton structure formed of a structural unit derived from a polymerizable monomer, wherein the skeleton structure includes a structure corresponding to a structure obtained by removing R² from a structural unit represented by the following formula (1), and wherein the antigen is carried on the surface of the latex particle by being bonded to a group represented by R² in the formula (1). The content of the structure corresponding to the structure obtained by removing R² from the structural unit represented by the formula (1) in the skeleton structure may be 5 mass% or more and less than 50 mass%. That is, the affinity particle may be an affinity particle obtained by treating the latex particle before the carriage of the antigen, the particle containing the polymer having the structural unit represented by the following formula (1), as follows: the group represented by R² in the formula (1) is caused to carry the antigen.

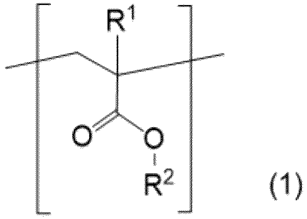

In the formula (1), R¹ represents a hydrogen atom or a methyl group, R² represents a group having an epoxy group, a group having a hydroxy group, or a group having a carboxy group, and R¹ and R² may each vary from structural unit to structural unit.

When the content of the structure corresponding to the structure obtained by removing R² from the structural unit represented by the formula (1) in the above-mentioned skeleton structure formed of the structural unit derived from the polymerizable monomers in the latex particle is 5 mass% or more, the hydrophobicity of the surface of the particle reduces, and hence nonspecific adsorption can be suppressed.

The structural unit represented by the formula (1) preferably includes a structural unit represented by the following formula (1-A). The structural unit represented by the formula (1-A) is preferred because the unit has one of a hydroxy group or a carboxy group, and is hence comparable or superior to a structure having an epoxy group in nonspecific adsorption-suppressing ability.

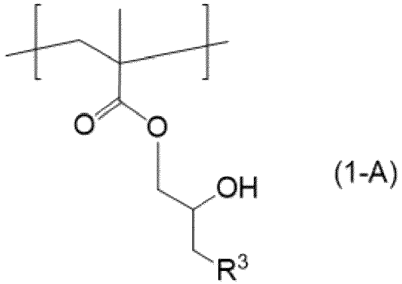

In the formula (1-A), R³ represents a hydroxy group, a group represented by the following formula (1-B), or a group represented by the following formula (1-C).

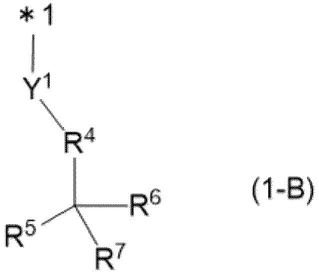

In the formula (1-B), R⁴ represents a single bond or a methylene group, R⁵, R⁶, and R⁷ each independently represent a hydrogen atom, a methyl group, a hydroxy group, or a hydroxymethyl group, and the group represented by the formula (1-B) has at least one hydroxy group, Y¹ represents a sulfur atom or an imino group, and *1 represents a bonding position.

At least one hydroxy group of the group represented by the formula (1-B) may be a hydroxy group in a hydroxymethyl group.

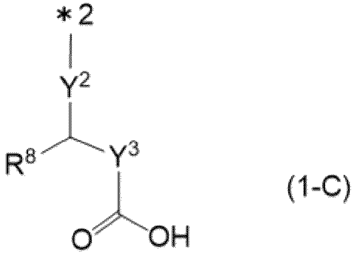

In the formula (1-C), R⁸ represents a hydrogen atom, a methyl group, a hydroxy group, or a carboxy group, Y² represents a sulfur atom or an imino group, Y³ represents a single bond or a methylene group, and *2 represents a bonding position.

A structure represented by the following formula (1-A-1), a structure represented by the following formula (1-A-2), a structure represented by the following formula (1-A-3), a structure represented by the following formula (1-A-4), a structure represented by the following formula (1-A-5), a structure represented by the following formula (1-A-6), a structure represented by the following formula (1-A-7), a structure represented by the following formula (1-A-8), a structure represented by the following formula (1-A-9), a structure represented by the following formula (1-A-10), a structure represented by the following formula (1-A-11), and a structure represented by the following formula (1-A-12) are represented below as specific examples of the structure represented by the formula (1-A), but the present invention is not limited thereto.

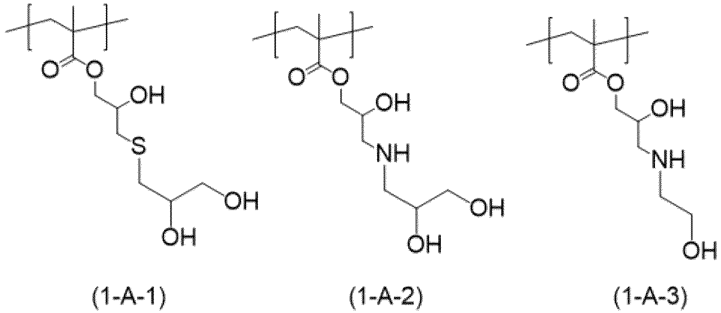

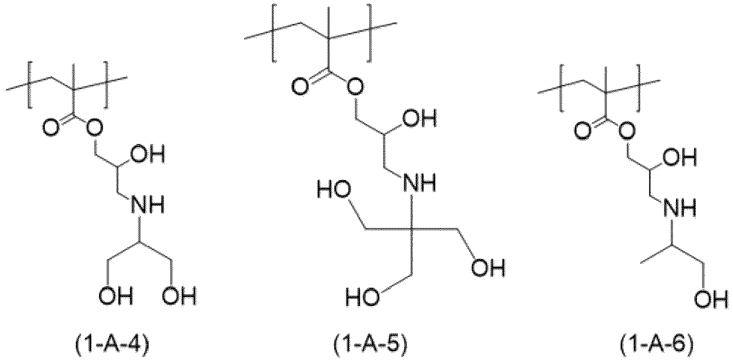

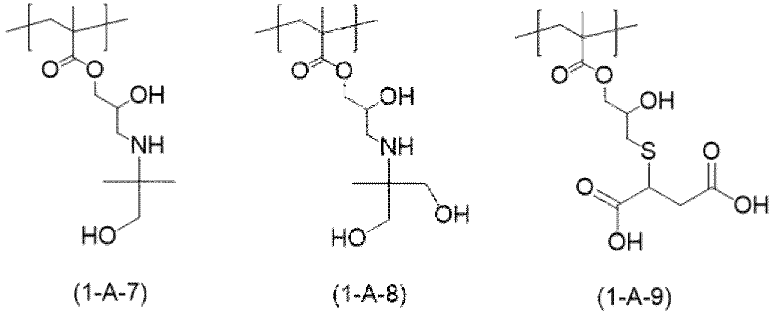

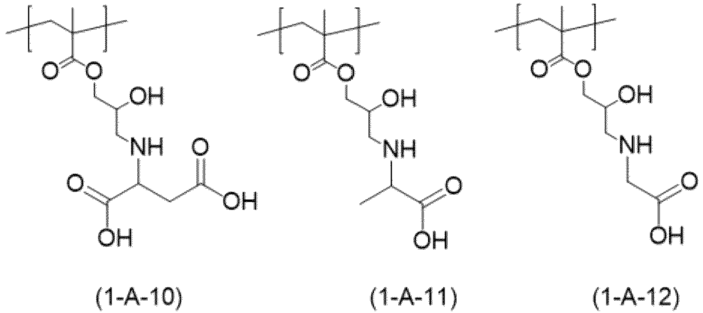

To obtain a latex particle before the carriage of the antigen, the particle containing the polymer having the structural unit represented by the formula (1) (in particular, the structural unit represented by the formula (1-A)), the surface of the latex particle before the carriage of the antigen may be subjected to surface treatment with a surface treatment agent. Examples of the surface treatment agent include mercaptosuccinic acid, 3-mercapto-1,2-propanediol, 3-amino-1,2-propanediol, and 2-amino-2-hydroxymethyl-1,3-propanediol.

### <Carriage of Antigen on Latex Particle>

In the present invention, the antigen for detecting the target immunoglobulin G may be carried on the surface of the latex particle through a chemical bond, or may be carried thereon by physical adsorption. However, the antigen is preferably carried through the chemical bond.

When the antigen is carried through the chemical bond, even in the case where the affinity particle is stored for a long time period under the state of being dispersed into the reagent liquid, the peeling of the antigen from the affinity particle and the denaturation of the antigen can be suppressed, and hence a liquid affinity particle-dispersed reagent that can be stably used over a long time period is easily obtained. In addition, the desorption and denaturation of the antigen can be suppressed even in a test reagent production process. For example, to remove an unreacted antigen and any other component that are unpreferred, the antigen and the component remaining in a reaction system, centrifugal washing with a washing liquid containing a surfactant or washing liquids different from each other in pH may be repeated in a step after the carriage of the antigen. Even in such case, the desorption and denaturation of the antigen hardly occur, and hence a step in accordance with purposes such as an improvement in detection sensitivity can be selected.

Although the carriage of the antigen through the chemical bond only needs to be performed in accordance with an ordinary method, a functional group of the latex particle before the carriage of the antigen and a primary amine, a secondary amine, a carboxy group, a thiol group, or the like of the antigen can be caused to react with each other. Examples of the functional group of the latex particle before the carriage of the antigen include a carboxy group, an amino group, an aldehyde group, an epoxy group, a thiol group, and a maleimide group. The latex particle before the carriage of the antigen may have only one kind of those functional groups, or may have two or more kinds thereof. It is preferred that the antigen be chemically bonded through the carboxy group of the side-chain structure represented by the formula (1-C) of the latex particle before the carriage of the antigen.

A conventionally known method may be applied as a method for the chemical reaction by which the carboxy group of the latex particle before the carriage of the antigen and the antigen are bonded to each other. For example, a carbodiimide-mediated reaction or an NHS ester activation reaction is a suitable case example of the chemical reaction. In addition, the following may be performed: avidin is bonded to the carboxy group, and a biotin-modified ligand (antigen) is bonded thereto.

Such an antigen in a dispersed state that the agglutination and multimerization of the molecules of the antigen are avoided is preferably used as the antigen to be carried from the viewpoints of a reduction in nonspecific adsorption and reactivity.

### <Antigen for detecting Target Immunoglobulin G>

In the present invention, the antigen to be carried on the surface of the latex particle for detecting the target immunoglobulin G is a polypeptide, which has, in a molecule thereof, an epitope sequence specifically recognized by the target immunoglobulin G and has a molecular weight of 10,000 or more. For example, a recombinant antigen produced by gene recombination (antigen expressed by introducing a gene encoding an antigen sequence into *Escherichia coli* or a mammalian cell), or an antigen extracted and purified from a human specimen may be used.

The use of the antigen having a molecular weight of 10,000 or more alleviates the possibility of the inhibition of an antigen-antibody reaction by a substance present near the surface of the latex particle. Further, coupled with the above-mentioned optimum range of the antigen-carrying amount, an intra-antigenic epitope can be caused to exist at an appropriate density on the surface of the particle. Accordingly, sufficient reactivity between the antigen and the target immunoglobulin G is obtained. In addition, an interparticle distance required for the agglutination of the particles through the immunoglobulin G widens to accelerate the particle agglutination.

In the present invention, two or more kinds of antigens may be used in combination. In that case, a product obtained by simultaneously causing a plurality of kinds of antigens to react with the latex particle may be used, or products obtained by carrying the respective antigens on the respective latex particles may be mixed later and used.

In the case of, for example, an affinity particle obtained by carrying a HCV antigen on the latex particle for detecting a HCV antibody, a plurality of kinds of epitope regions are preferably used so as to be caused to react with many kinds of HCV antibodies in a human specimen. An antigen having the very amino acid sequence of a HCV virus may be used. Alternatively, a chimeric antigen combined with an epitope region may be used. To maintain the reactivity and dispersed state of the antigen, the antigen may be treated with a reducing agent, a surfactant, or the like before being carried on the latex particle.

In the case of an affinity particle obtained by carrying a HBc antigen on the latex particle for detecting a HBc antibody, an antigen including the full-length sequence of a HBV virus core protein (HBc) may be used, or an antigen including the main epitope thereof may be used. In addition, treatment with a surfactant or the like may be performed as pretreatment.

### <Test Reagent and Detection Method>

A test reagent according to the present invention may be a test reagent including a first reagent liquid having dispersed thereinto the affinity particle according to the present invention.

Although the test reagent according to the present invention may be a test reagent including one or two or more liquids, a test reagent including two liquids out of such reagents is preferred from the viewpoint of measurement in a general-purpose biochemical apparatus.

The test reagent including two or more liquids is, for example, a test reagent including a "liquid (first reagent liquid) having dispersed thereinto an affinity particle having carried thereon a protein (antigen)" and a "buffer solution (second reagent liquid)."

In this case, the following affinity particle according to the present invention is dispersed into the liquid (first reagent liquid) having dispersed thereinto the affinity particle: the affinity particle has a volume-average particle diameter of 400 nm or less; the affinity particle includes the latex particle and the protein carried on the surface of the latex particle; the protein contains the antigen having a molecular weight of 10,000 or more; and the amount of the protein carried on the surface of the latex particle is 1.0 µg or more and 20.0 µg or less per 1 mg of the affinity particle. In addition, the buffer solution (second reagent liquid) may be free of the above-mentioned affinity particle.

An example of a detection method for detecting immunoglobulin G in a human specimen by a latex agglutination method according to the present invention, the detection method including using the test reagent including two or more liquids, is described.

That is, the detection method is a detection method for detecting immunoglobulin G in a human specimen by a latex agglutination method through use of a test reagent including a first reagent liquid having dispersed thereinto an affinity particle and a second reagent liquid that is a buffer solution, the detection method including: a first step of obtaining a mixed liquid containing the human specimen and the buffer solution (second reagent liquid); a second step of mixing the mixed liquid obtained in the first step and the liquid (first reagent liquid) having dispersed thereinto the affinity particle to cause the mixed liquid and the first reagent liquid to react with each other, to thereby provide a reaction liquid; and a third step of measuring a concentration of the immunoglobulin G in the reaction liquid, wherein the affinity particle has a volume-average particle diameter of 400 nm or less, wherein the affinity particle includes a latex particle and a protein carried on a surface of the latex particle, wherein the protein contains an antigen having a molecular weight of 10,000 or more for detecting the immunoglobulin G, and wherein an amount of the protein carried on the surface of the latex particle is 1.0 µg or more and 20.0 µg or less per 1 mg of the affinity particle.

In the above-mentioned first step, the mixed liquid may be obtained by mixing the human specimen and the buffer solution (second reagent liquid). The reaction in the above-mentioned second step is preferably performed in the pH range of from 3.0 to 11.0. In addition, a mixing temperature falls within the range of from 20°C to 50°C, and a mixing time falls within the range of from 1 minute to 20 minutes. The concentration of the affinity particle in the reaction liquid is preferably from 0.001 mass% to 5 mass%, more preferably from 0.01 mass% to 1 mass%.

In the detection method according to the present invention, interparticle agglutination caused as a result of the mixing of the affinity particle according to the present invention and the specimen is optically detected. Thus, the target immunoglobulin G in the specimen can be detected. As a method of optically detecting an agglutination reaction between the affinity particles, the amount of a change in, for example, scattered light intensity, transmitted light intensity, or absorbance only needs to be measured with an optical instrument that can detect such value.

The test reagent according to the present invention preferably includes: a nonionic surfactant and an amphoteric surfactant, and further includes at least one metal salt selected from sodium chloride, potassium chloride, and magnesium chloride. In addition, the test reagent according to the present invention may be a test reagent including the first reagent liquid having dispersed thereinto the affinity particle according to the present invention and a second reagent liquid that is a buffer solution, in which the second reagent liquid is free of the affinity particle according to the present invention, contains the nonionic surfactant and the amphoteric surfactant, and further contains the at least one metal salt selected from sodium chloride, potassium chloride, and magnesium chloride. The test reagent according to the present invention may be a test reagent, which includes at least one of the nonionic surfactant and the amphoteric surfactant, and further includes the at least one metal salt selected from sodium chloride, potassium chloride, and magnesium chloride.

In addition, the test reagent according to the present invention may be a test reagent, which includes the nonionic surfactant, and further includes the at least one metal salt selected from sodium chloride, potassium chloride, and magnesium chloride.

### <Surfactant>

The test reagent according to the present invention preferably includes the nonionic surfactant and the amphoteric surfactant.

In the case of a test reagent including two liquids, those components may be incorporated into one of the buffer solution (second reagent liquid) or the liquid (first reagent liquid) having dispersed thereinto the affinity particle, or may be incorporated into both the liquids.

A primary object of the use of the nonionic surfactant and/or the amphoteric surfactant is to alleviate nonspecific agglutination due to a substance except the target substance present in the human specimen.

The nonionic surfactant is a surfactant whose polar moiety is nonionic (not charged), and the surfactant has been known to have a suppressing effect on an interaction between a lipid and a protein. The use of the nonionic surfactant in the test reagent according to the present invention improves the dispersibility of the affinity particle (moderately weakens the agglutination property thereof). Further, the nonspecific adsorption of the substance except the target substance to the surface of the affinity particle (in particular, a hydrophobic component such as polystyrene) is alleviated, and hence the detection amounts of a negative specimen group can be brought close to 0.

Examples of the nonionic surfactant include a polyoxyethylene sorbitan fatty acid ester (trademark: Tween 20, Tween 80, or the like), a polyoxyethylene octylphenyl ether (trademark: Triton X-100, Triton X-114, or the like), a polyoxyethylene alkyl ether (trademark: Brij 35, Brij 58, or the like), an alkyl glycoside (n-octyl-β-D-glucopyranoside, n-octyl-β-D-thioglucoside, n-dodecyl-β-D-maltoside, or the like), and an N-D-gluco-N-methylalkanamide (MEGA 9, MEGA 10, or the like). Of those, polyoxyethylene sorbitan monolaurate (trademark: Tween 20) and n-octyl-β-D-glucopyranoside are preferred.

The content of the nonionic surfactant is preferably as follows: the surfactant is incorporated into the test reagent in such an amount that its concentration becomes equal to or more than a critical micelle concentration in a step of detecting the immunoglobulin G in the human specimen. The content of the nonionic surfactant in the reaction liquid in the above-mentioned third step (i.e., the amount thereof with respect to a total including, for example, the human specimen, the buffer solution (second reagent liquid), and the liquid (first reagent liquid) having dispersed thereinto the affinity particle) is preferably as described below. In the case of polyoxyethylene sorbitan monolaurate (Tween 20), the above-mentioned content is preferably 0.005 mass% or more and 5 mass% or less, more preferably 0.03 mass% or more and 0.5 mass% or less. In the case of n-octyl-β-D-glucopyranoside, the above-mentioned content is preferably 0.3 mass% or more and 5 mass% or less, more preferably 0.5 mass% or more and 2 mass% or less. The amount of Tween 20 with respect to the total of the buffer solution (second reagent liquid) and the liquid (first reagent liquid) having dispersed thereinto the affinity particle is preferably 0.006 mass% or more and 5.6 mass% or less, more preferably 0.03 mass% or more and 0.6 mass% or less.

The amphoteric surfactant has, in its polar moiety, both ions that are positively and negatively charged. Unlike the nonionic surfactant, the amphoteric surfactant has been known to have the effect by which some interprotein interactions can be suppressed. The use of the amphoteric surfactant in the test reagent according to the present invention can alleviate the nonspecific adsorption of the substance except the target substance to the antigen carried mainly on the latex particle to bring the detection amounts of the negative specimen group close to 0. While the amphoteric surfactant has an action stronger than that of the nonionic surfactant, the amphoteric surfactant has a weak denaturing action on a protein itself like an anionic surfactant and a cationic surfactant. Accordingly, the amphoteric surfactant is effective in alleviating the nonspecific adsorption without largely impairing the reactivity of the antibody important for the detection.

Examples of the amphoteric surfactant include 3-[(3-cholamidopropyl)dimethylammonio]-1-propanesulfonate (hereinafter also referred to as "CHAPS"), 3-[(3-cholamidopropyl)dimethylammonio]-2-hydroxysulfonate (hereinafter also referred to as "CHAPSO"), n-dodecyl-N,N-dimethyl-3-ammonio-1-propanesulfonate (Zwittergent 3-12), and N,N-dimethyldodecylamine N-oxide. Of those, 3-[(3-cholamidopropyl)dimethylammonio]-1-propanesulfonate (CHAPS) and 3-[(3-cholamidopropyl)dimethylammonio]-2-hydroxysulfonate (CHAPSO) are preferred.

The content of the amphoteric surfactant in the reaction liquid in the above-mentioned third step (i.e., the amount thereof with respect to a total including the human specimen, the buffer solution (second reagent liquid), and the liquid (first reagent liquid) having dispersed thereinto the affinity particle) is preferably as described below. The content is preferably 0.05 mass% or more and 5.0 mass% or less, more preferably 0.1 mass% or more and 3.0 mass% or less. The amount of the amphoteric surfactant with respect to the total of the buffer solution (second reagent liquid) and the liquid (first reagent liquid) having dispersed thereinto the affinity particle is preferably 0.06 mass% or more and 5.6 mass% or less, more preferably 0.1 mass% or more and 3.3 mass% or less.

### <Metal salt>

The test reagent of the present invention preferably includes at least one metal salt selected from sodium chloride, potassium chloride, and magnesium chloride.

In the case of a test reagent including two or more liquids, the above-mentioned metal salt may be incorporated into one of the buffer solution (second reagent liquid) or the liquid (first reagent liquid) having dispersed thereinto the affinity particle, or may be incorporated into each of both the liquids. However, the buffer solution (second reagent liquid) preferably contains the above-mentioned metal salt.

In each of the test reagent and the detection method according to the present invention, it is preferred that the buffer solution (second reagent liquid) contain at least one metal salt selected from sodium chloride, potassium chloride, and magnesium chloride, and that the total content of the at least one metal salt selected from sodium chloride, potassium chloride, and magnesium chloride in the buffer solution (second reagent liquid) be 120 mM or more and 500 mM or less. In addition, in each of the test reagent and the detection method according to the present invention, it is preferred that at least one of the first reagent liquid and the second reagent liquid contain at least one metal salt selected from sodium chloride, potassium chloride, and magnesium chloride, and that the total content of the at least one metal salt selected from sodium chloride, potassium chloride, and magnesium chloride with respect to the total of the first reagent liquid and the second reagent liquid be 90 mM or more and 400 mM or less. In addition, the detection method according to the present invention is preferably as follows: in the third step of measuring the concentration of the immunoglobulin G in the reaction liquid obtained by mixing the mixed liquid containing the human specimen and the buffer solution (second reagent liquid), and the liquid (first reagent liquid) having dispersed thereinto the affinity particle to cause the mixed liquid and the first reagent liquid to react with each other, the reaction liquid contains at least one metal salt selected from sodium chloride, potassium chloride, and magnesium chloride, the at least one metal salt not being derived from the human specimen; and the total content of the at least one metal salt selected from sodium chloride, potassium chloride, and magnesium chloride, the at least one metal salt not being derived from the human specimen, in the reaction liquid in the third step is 80 mM or more and 360 mM or less. In addition, the test reagent according to the present invention may be a test reagent including the first reagent liquid having dispersed thereinto the affinity particle and the second reagent liquid that is the buffer solution, in which the second reagent liquid is free of the affinity particle, contains the nonionic surfactant and the amphoteric surfactant, and further contains the at least one metal salt selected from sodium chloride, potassium chloride, and magnesium chloride, and in which the total content of the at least one metal salt selected from sodium chloride, potassium chloride, and magnesium chloride in the second reagent liquid is 120 mM or more and 500 mM or less.

When the amount of the metal salt derived from the test reagent is set within the above-mentioned ranges, the electric conductivity of a measuring system moderately increases. Accordingly, when an affinity particle having surface charge is used, the electrostatic nonspecific adsorption of a substance that is not the target substance present in the human specimen to the affinity particle is alleviated, and hence a variation between the detection amounts of the negative specimen group can be suppressed. In addition, when an electric double layer on the surface of the affinity particle is made moderately thin, a particle agglutination property is improved, and hence detection sensitivity can be improved.

When the test reagent includes an ionic substance in addition to the above-mentioned metal salt, in the third step of detecting the immunoglobulin G, an electric conductivity derived from the ionic substances in the test reagent including the above-mentioned metal salt is preferably set to 15 mS/cm or more and 60 mS/cm or less.

### <Other Component>

The test reagent according to the present invention may include a substance, such as a buffering agent, a sensitizer, a surfactant, a saccharide, an albumin, or immunoglobulin G, to the extent that the object of the present invention can be achieved.

In the case of a test reagent including two or more liquids, those components may each be incorporated into one of the buffer solution (second reagent liquid) or the liquid (first reagent liquid) having dispersed thereinto the affinity particle, or may be incorporated into each of both the liquids.

At least the buffer solution (second reagent liquid) contains a buffering agent. Examples of the buffer solution containing the buffering agent include various aqueous buffer solutions, such as a phosphate buffer solution, a glycine buffer solution, a Good's buffer solution, a Tris buffer solution, a HEPES buffer solution, a MES buffer solution, and an ammonia buffer solution. However, the buffer solution in the test reagent according to the present invention is not limited thereto.

Examples of the sensitizer for latex agglutination measurement include, but not limited to, polyvinyl alcohol, polyvinylpyrrolidone, polyethylene glycol, and alginic acid.

### [Method of calculating Amount of Protein carried on Surface of Latex Particle]

The amount (µg) of the protein carried on the surface of the latex particle in the affinity particle (amount of the protein carried on the surface of the latex particle per 1 mg of the affinity particle) may be determined as the amount of the protein in terms of bovine serum albumin (BSA).

First, the affinity particle is sedimented by centrifuging the reagent liquid having dispersed thereinto the affinity particle, and the supernatant is removed. Next, pure water is added to disperse the affinity particle. Centrifugation is repeated again to provide a sedimented particle component (referred to as "particle component 1"). The dry weight of the component after its drying at 130°C is measured, and is defined as the mass "W" of the latex particle (affinity particle) having carried thereon the protein.

Similarly, the "particle component 1" is separately collected from the same amount of the reagent liquid. The component is dispersed in pure water again, and a dispersion (referred to as "particle aqueous dispersion 1") is prepared by using the particle mass "W" measured as described above so that its particle concentration may be 0.2 mass%. Protein determination is performed by the following method.

First, the liquid A and liquid B of a protein assay BCA kit (Wako Pure Chemical Industries, Ltd.) are mixed at a ratio "liquid A:liquid B" of 50:1 (v/v), and the prepared liquid is defined as a liquid AB. 200 Microliters of the liquid AB is added to 25 mg (particle amount: 50 µg) of the "particle aqueous dispersion 1" (0.2 mass% solution), and the mixture is incubated at 60°C. The solution is centrifuged, and the supernatant is loaded into a 96-well microplate. The absorbance of the supernatant at 562 nm is measured with a microplate reader together with standard protein samples (several 0 µg/mL to 200 µg/mL solutions obtained by diluting a bovine serum albumin (BSA) standard with pure water), and the amount of the protein in terms of BSA is calculated from a standard curve.

The amount (µg) of the protein carried on the surface of the latex particle per 1 mg of the affinity particle is determined by dividing the calculated amount of the protein in terms of BSA by a particle mass (0.05 mg when the amount is evaluated with 25 mg of the 0.2 mass% solution as described above).

### [Method of measuring Volume-average Particle Diameter of Affinity Particle]

The volume-average particle diameter of the affinity particle is measured under a state in which the reagent liquid containing the affinity particle is diluted with ion-exchanged water (having an electric conductivity of 10 µS/cm or less). A dynamic light scattering method is applied to the measurement of the volume-average particle diameter. Specifically, the measurement is performed with, for example, a zeta sizer (ZETA SIZER Nano-ZS, ZETA SIZER Ultra, or the like: Spectris Co., Ltd.) at 25°C. With regard to analysis parameters, the refractive index of latex (n≈1.59) is selected as the refractive index of the particle, and pure water is selected as a solvent. The measurement is performed three times, and the average of the measured values of the volume-average particle diameter is adopted.

### [Examples]

The present invention is described in detail below by way of Examples. However, the present invention is by no means limited to these Examples.

### [Synthesis of Latex Particles 1]

### (Step-1/Production of Core Particles A)

23.5 Grams of styrene (hereinafter also referred to as "St": Kishida Chemical Co., Ltd.), 0.43 g of divinylbenzene (hereinafter also referred to as "DVB": Kishida Chemical Co., Ltd.), and 800 g of ion-exchanged water were weighed in a 2-liter four-necked separable flask to provide a mixed liquid. The mixed liquid was held at 70°C while being stirred at 100 rpm, and oxygen was removed from the inside of the above-mentioned four-necked separable flask by flowing nitrogen at a flow rate of 200 ml/min. Next, a dissolved liquid separately prepared by dissolving 1.02 g of V-50 (FUJIFILM Wako Pure Chemical Corporation) in 50 g of ion-exchanged water was added to the above-mentioned mixed liquid to start soap-free emulsion polymerization. The liquids were caused to react with each other for 48 hours from the start of the polymerization to provide a dispersion of core particles A each having a copolymer of St and DVB. Part of the dispersion was collected, and the volume-average particle diameter of the core particles A was evaluated. As a result, the volume-average particle diameter was 280 nm.

### (Step-2/Production of Mother Particles A)

The amount of the dispersion of the core particles A having a solid content concentration of 2.0% was adjusted to 150 g with ion-exchanged water. Next, 1.54 g of glycidyl methacrylate (hereinafter also referred to as "GMA": Kishida Chemical Co., Ltd.) was added to the dispersion. The mixture was held at 70°C while being stirred at 100 rpm, and oxygen was removed from the inside of the above-mentioned four-necked separable flask by flowing nitrogen at a flow rate of 200 ml/min. Then, a dissolved liquid separately prepared by dissolving 0.018 g of V-50 (FUJIFILM Wako Pure Chemical Corporation) in 1 g of ion-exchanged water was added to the above-mentioned mixture to start the formation of a shell. The mixture was continuously stirred for 17 hours after the start of the reaction to provide a dispersion containing mother particles A each having a core-shell structure. The above-mentioned dispersion was slowly cooled to room temperature, and then part of the dispersion was collected and evaluated for its polymerization conversion ratio by using gas chromatography. As a result, it was recognized that the ratio was substantially 100%. The above-mentioned polymerization conversion ratio was calculated from the amounts of the monomers loaded in the polymerization step. The solution after the polymerization reaction was analyzed by using gas chromatography or the like, and the amount of the remaining monomer was determined, followed by the determination of the value (polymerization conversion ratio) of the extent to which the monomers were polymerized and converted by the polymerization reaction from the amount of the remaining monomer. In this case, when the amount of the remaining monomer was equal to or less than a detection limit, it was judged that the polymerization conversion ratio was substantially 100% (all the monomers polymerized).

### (Step-3/Production of Latex Particles 1)

The following material was added to the aqueous dispersion containing the mother particles A, and triethylamine (Kishida Chemical Co., Ltd.) was further added to the mixture to adjust its pH to 10.
·An aqueous solution prepared in advance by dissolving mercaptosuccinic acid (hereinafter also referred to as "MSA": Wako Pure Chemical Industries, Ltd.) and 3-mercapto-1,2-propanediol (hereinafter also referred to as "3MPD": Wako Pure Chemical Industries, Ltd.) (MSA:3MPD=7:3 (mole fraction), and the total number of moles of MSA and 3MPD was twice as large as the number of moles of GMA used in Step-2)

Next, while the above-mentioned mixture was stirred at 200 rpm, its temperature was increased to 70°C. Further, the mixture was subjected to surface treatment by being held under the state for 18 hours. Thus, a dispersion of latex particles 1 was obtained. The latex particles 1 were separated from the above-mentioned dispersion by centrifugal separation, and the latex particles 1 were redispersed in ion-exchanged water. The foregoing operation was repeated eight times to purify the latex particles 1. Finally, the latex particles 1 were stored under the state of an aqueous dispersion in which their concentration was adjusted to 1.0 mass%. The volume-average particle diameter of the latex particles 1 was evaluated. As a result, the volume-average particle diameter was 320 nm. The results of the evaluations of the physical properties of the latex particles 1 are shown in Table 1.

### [Synthesis of Latex Particles 2]

A dispersion of latex particles 2 was obtained by the same experiment operation as that of Example 1 except that: the mother particles A produced in Example 1 were used; and 3-amino-1,2-propanediol (hereinafter also referred to as "3APD": Tokyo Chemical Industry Co., Ltd.) was used instead of 3MPD in Step-3 of Example 1. The results of the evaluations of the physical properties of the latex particles 2 are shown in Table 1.

### [Synthesis of Latex Particles 3]

The mother particles A produced in Example 1 were used, and 2-amino-2-hydroxymethyl-1,3-propanediol (tris(hydroxymethyl)aminomethane, hereinafter also referred to as "Tris": Kishida Chemical Co., Ltd.) was used instead of 3MPD in Step-3 of Example 1. A dispersion of latex particles 3 was obtained by the same experiment operation as that of Example 1 except the foregoing. The results of the evaluations of the physical properties of the latex particles 3 are shown in Table 1.

### [Synthesis of Latex Particles 4]

In Step-1 of Example 1, the usage amount of St was changed to 71.75 g, the usage amount of DVB was changed to 1.30 g, the usage amount of ion-exchanged water was changed to 1,190.7 g, and the usage amount of V-50 was changed to 3.11 g, and the number of revolutions of the stirring was changed from 100 rpm to 140 rpm. A dispersion of core particles B was obtained by the same experiment operation as that of Example 1 except the foregoing.

Next, mother particles B were produced, and then a dispersion of latex particles 4 was obtained by the same experiment operations as those of Step-2 and Step-3 of Example 1 except that the core particles B were used. The results of the evaluations of the physical properties of the latex particles 4 are shown in Table 1.

### [Synthesis of Latex Particles 5]

### (Production of Mother Particles C)

The following materials were weighed in a 2-liter four-necked separable flask to provide a mixed liquid.
·12.0 Grams of styrene (St: Kishida Chemical Co., Ltd.)
·18.0 Grams of glycidyl methacrylate (GMA: Kishida Chemical Co., Ltd.)
·0.45 Gram of divinylbenzene (DVB: Kishida Chemical Co., Ltd.)
·1,200 Grams of ion-exchanged water

The mixed liquid was held at 70°C while being stirred at 200 rpm, and oxygen was removed from the inside of the above-mentioned four-necked separable flask by flowing nitrogen at a flow rate of 200 ml/min. Next, a dissolved liquid separately prepared by dissolving 0.68 g of V-50 (FUJIFILM Wako Pure Chemical Corporation) in 30 g of ion-exchanged water was added to the above-mentioned mixed liquid to start soap-free emulsion polymerization. Two hours after the start of the polymerization, 3.0 g of GMA was further added to the resultant, and the mixture was subjected to a reaction for 20 hours to provide a dispersion of mother particles C each having a copolymer of St, GMA, and DVB. The above-mentioned dispersion was slowly cooled to room temperature, and then part of the dispersion was collected and evaluated for its polymerization conversion ratio by using gas chromatography. As a result, it was recognized that the ratio was substantially 100%.

### (Production of Latex Particles 5)

In Step-3 of Example 1, the mother particles C were used instead of the mother particles A, the ratio "MSA:3MPD" was changed from 7:3 to 2:8 (mole fraction), and the total number of moles of MSA and 3MPD was changed so as to be equal to the number of moles of GMA used in the production of the mother particles C. A dispersion of latex particles 5 was obtained by the same experiment operation as that of Step-3 of Example 1 except the foregoing. The results of the evaluations of the physical properties of the latex particles 5 are shown in Table 1.

### [Synthesis of Comparative Particles 1]

A dispersion of core particles D was obtained by the same experiment operation as that of Example 1 except that in Step-1 of Example 1, the usage amount of St was changed to 99.40 g, the usage amount of DVB was changed to 1.80 g, the usage amount of ion-exchanged water was changed to 1,150.15 g, and the usage amount of V-50 was changed to 4.31 g, and the number of revolutions of the stirring was changed from 100 rpm to 200 rpm.

After that, mother particles D and comparative particles 1 were obtained by the same experiment operations as those of Step-2 and Step-3 of Example 1 except that the core particles D were used. The results of the evaluations of the physical properties of the comparative particles 1 are shown in Table 1.

**Table 1**

| | Core particles | Mother particles | Latex particles before carriage of antigen | | | |
|---|---|---|---|---|---|---|
| | | | Content of structure corresponding to structure obtained by removing R² from structural unit represented by formula (1) in skeleton structure of latex particles (mass%) | Surface treatment agent | | Average particle diameter |
| | | | | Molar ratio "carboxy group-containing compound:hydroxy group-containing compound" | Usage amount with respect to GMA | |
| Latex particles 1 | A | A | 24 | MSA:3MPD=7:3 | 2 mole times | 320 nm |
| Latex particles 2 | | | 24 | MSA:3APD=7:3 | | 308 nm |
| Latex particles 3 | | | 24 | MSA:Tris=7:3 | | 313 nm |
| Latex particles 4 | B | B | 24 | Same as latex particles 1 | 2 mole times | 370 nm |
| Latex particles 5 | - | C | 49 | MSA:3MPD=2:8 | Equimolar amount | 233 nm |
| Comparative particles 1 | D | D | 24 | Same as latex particles 1 | 2 mole times | 560 nm |

### [Example 1 (Test Reagent 1)]

A test reagent 1 is formed of two liquids, that is, a liquid (first reagent liquid) 1 having dispersed thereinto affinity particles and a buffer solution (second reagent liquid) A.

### (Production of Liquid 1 having dispersed thereinto Affinity Particles)

The solvent substitution of a HCV antigen solution (manufactured by TRINA BIOREACTIVES AG) was performed with an ultrafiltration device (Amicon Ultra 10K: Merck Millipore). After that, it was recognized by SDS polyacrylamide gel electrophoresis (SDS-PAGE) that the antigen was a protein having a molecular weight of 10,000 or more. Next, the concentration of the protein in the antigen solution was measured by the method described in the above-mentioned section [Method of calculating Amount of Protein carried on Surface of Latex Particle] with a protein assay BCA kit (Wako Pure Chemical Industries, Ltd.) and a BSA standard protein.

The solution was diluted so that its protein concentration became 0.14 mg/mL. Thus, a "HCV antigen solution R" was obtained. A 10 mM HEPES buffer solution having added thereto 20 mM (±) dithiothreitol (DTT: FUJIFILM Wako Pure Chemical Corporation) was used as a diluent.

60 Microliters of a 1.7 mass% aqueous suspension of the latex particles 1 was dispensed into a microtube. 30 Microliters of a 0.1% aqueous solution of 1-(3-dimethylaminopropyl)-3-ethylcarbodiimide hydrochloride and 30 µL of a 0.1% aqueous solution of N-hydroxysulfosuccinimide sodium were added thereto. The above-mentioned mixture was stirred at room temperature for 30 minutes to provide a dispersion of particles whose carboxy groups were activated (activated particle dispersion).

After the dispersion had been subjected to centrifugal washing, 50 µL of a 10 mM HEPES buffer solution was added thereto, and the particles whose carboxy groups were activated were dispersed with an ultrasonic wave. 50 Microliters of the "HCV antigen solution R" (0.14 mg/mL solution) was added thereto (7.0 µg of the HCV antigen was brought into contact with 1 mg of the particles), and the mixture was stirred at room temperature for 1 hour so that the antigen was bonded to each of the carboxy groups of the particles.

After the resultant had been subjected to centrifugal washing, 240 µL of a masking buffer solution (0.3 M glycine solution containing 0.1% Tween 20, pH: 8.0) was added thereto, and the mixture was stirred at room temperature for 1 hour. After that, the mixture was left at rest at 4°C overnight so that glycine was bonded to each of the remaining activated carboxy groups. Thus, the affinity particles were obtained.

After the resultant had been subjected to centrifugal washing, 0.5 mL of a storage buffer solution (10 mM HEPES containing 0.01% Tween 20, pH: 7.9) was added thereto, and the particles were dispersed with an ultrasonic wave. Thus, the liquid 1 having dispersed thereinto the affinity particles was obtained.

The volume-average particle diameter of the affinity particles dispersed into the liquid 1 having dispersed thereinto the affinity particles was evaluated. As a result, the volume-average particle diameter was 318 nm. In addition, the amount of the protein carried on the surfaces of the latex particles of the liquid 1 having dispersed thereinto the affinity particles was evaluated. As a result, the amount was 6.4 µg per 1 mg of the affinity particles.

### (Buffer Solution A)

The following materials were mixed and dissolved in ultrapure water so as to have concentrations described below, and the pH of the solution was adjusted to 7.5. The resultant solution was defined as the buffer solution A.
·100 mM HEPES
·500 mM sodium chloride
·0.1 mass% Tween 20
·0.5 mass% 3-[(3-cholamidopropyl)dimethylammonio]-1-propanesulfonate (CHAPS)
·0.2 mass% polyethylene glycol (molecular weight: about 500,000)

Thus, the test reagent 1 formed of two liquids, that is, the liquid 1 having dispersed thereinto the affinity particles and the buffer solution A was obtained. The results of the evaluations of the physical properties of the test reagent 1 are shown in Tables 2-1 and 2-2.

### [Examples 2 and 3 (Test Reagents 2 and 3)]

The protein concentration of the "HCV antigen solution R" used in the section (Production of Liquid 1 having dispersed thereinto Affinity Particles) of Example 1 was changed from 0.14 mg/mL to 0.04 mg/mL or 0.40 mg/mL, respectively (2.0 µg or 20.0 µg of the HCV antigen was brought into contact with 1 mg of the particles, respectively). A liquid 2 having dispersed thereinto affinity particles and a liquid 3 having dispersed thereinto affinity particles were each produced in the same manner as in Example 1 except the foregoing. Thus, a test reagent 2 formed of two liquids, that is, the liquid 2 having dispersed thereinto the affinity particles and the buffer solution A was obtained. In addition, a test reagent 3 formed of the liquid 3 having dispersed thereinto the affinity particles and the buffer solution A was obtained. The results of the evaluations of the physical properties of the test reagent 2 and the test reagent 3 are shown in Tables 2-1 and 2-2.

### [Examples 4 to 7 (Test Reagents 4 to 7)]

Liquids 4 to 7 having dispersed thereinto affinity particles were each produced in the same manner as in Example 1 except that the latex particles 1 used in the section (Production of Liquid 1 having dispersed thereinto Affinity Particles) of Example 1 were changed to the latex particles 2 to 5, respectively. Thus, a test reagent 4 formed of two liquids, that is, the liquid 4 having dispersed thereinto the affinity particles and the buffer solution A, a test reagent 5 formed of two liquids, that is, the liquid 5 having dispersed thereinto the affinity particles and the buffer solution A, a test reagent 6 formed of two liquids, that is, the liquid 6 having dispersed thereinto the affinity particles and the buffer solution A, and a test reagent 7 formed of two liquids, that is, the liquid 7 having dispersed thereinto the affinity particles and the buffer solution A were obtained. The results of the evaluations of the physical properties of the test reagents 4 to 7 are shown in Tables 2-1 and 2-2.

### [Examples 8 and 9 (Test Reagents 8 and 9)]

The following buffer solution B and the following buffer solution C were produced.

### (Buffer Solution B)

A solution obtained by mixing the following materials in ultrapure water so as to have concentrations described below was defined as the buffer solution B.
·PBS(-) (pH: 7.4, containing sodium chloride having a concentration of 137 mM with respect to the buffer solution B)
·0.1 mass% Tween 20
·0.5 mass% CHAPS
·0.2 mass% polyethylene glycol (molecular weight: about 500,000)

### (Buffer Solution C)

A buffer solution C was produced in the same manner as in the buffer solution A except that in the buffer solution A, 0.5 mass% CHAPS was changed to 0.02 mass% sodium dodecyl sulfate (SDS).

Thus, a test reagent 8 formed of two liquids, that is, the liquid 1 having dispersed thereinto the affinity particles and the buffer solution B, and a test reagent 9 formed of two liquids, that is, the liquid 1 having dispersed thereinto the affinity particles and the buffer solution C were obtained.

### [Comparative Examples 1 and 2 (Comparative Reagents 1 and 2)]

Liquids 8 and 9 having dispersed thereinto affinity particles were each produced in the same manner as in Example 1 except that the protein concentration of the "HCV antigen solution R" used in the section (Production of Liquid 1 having dispersed thereinto Affinity Particles) of Example 1 was changed from 0.14 mg/mL to 0.60 mg/mL or 0.018 mg/mL, respectively (30.0 µg or 0.9 µg of the antigen was brought into contact with 1 mg of the particles, respectively). Thus, a comparative reagent 1 formed of two liquids, that is, the liquid 8 having dispersed thereinto the affinity particles and the buffer solution A, and a comparative reagent 2 formed of two liquids, that is, the liquid 9 having dispersed thereinto the affinity particles and the buffer solution A were obtained. The results of the evaluations of the physical properties of the comparative reagents 1 and 2 are shown in Tables 2-1 and 2-2.

### [Comparative Example 3 (Comparative Reagent 3)]

A liquid 10 having dispersed thereinto affinity particles was produced in the same manner as in Example 1 except that the latex particles 1 used in the section (Production of Liquid 1 having dispersed thereinto Affinity Particles) of Example 1 were changed to the comparative particles 1. Thus, a comparative reagent 3 formed of two liquids, that is, the liquid 10 having dispersed thereinto the affinity particles and the buffer solution A was obtained. The results of the evaluations of the physical properties of the comparative reagent 3 are shown in Tables 2-1 and 2-2.

### [Comparative Example 4 (Comparative Reagent 4)]

### (Production of Liquid 11 having dispersed thereinto Affinity Particles)

A 0.14 mg/mL "HCV antigen solution R" was obtained by the same experiment operation as that of the section (Production of Liquid 1 having dispersed thereinto Affinity Particles).

The latex particles 1 used in the liquid 1 having dispersed thereinto the affinity particles were changed to carboxy group-containing polystyrene particles (product name: "FUJIKURA LATEX FK-C400D": manufactured by Fujikura Kasei Co., Ltd.). The antigen was bonded to each of the carboxy groups of the particles, and then glycine was bonded to each of the remaining activated carboxy groups by the same experiment operation as that of the section (Production of Liquid 1 having dispersed thereinto Affinity Particles) except the foregoing.

The resultant was repeatedly subjected to centrifugal washing with a storage buffer solution (50 mM HEPES containing 3% BSA and 0.05% Tween 20, pH: 7.6), and then 0.5 mL of the storage buffer solution was added to disperse the particles. Thus, a liquid 11 having dispersed thereinto affinity particles was obtained.

Thus, a comparative reagent 4 formed of two liquids, that is, the liquid 11 having dispersed thereinto the affinity particles and the buffer solution A was obtained.

**Table 2-1**

| | Liquid having dispersed thereinto affinity particles | | | | | |
|---|---|---|---|---|---|---|
| | | Latex particles | Loading amount of HCV antigen per 1 mg of affinity particles (µg) | Protein except antigen | Volume-average particle diameter of affinity particles | Amount of protein carried on surfaces of particles (µg/mg) |
| Test reagent 1 | 1 | Latex particles 1 | 7.0 | - | 318 nm | 6.4 |
| Test reagent 2 | 2 | | 2.0 | - | 308 nm | 1.9 |
| Test reagent 3 | 3 | | 20.0 | - | 329 nm | 16.8 |
| Test reagent 4 | 4 | Latex particles 2 | 7.0 | | 314 nm | 5.5 |
| Test reagent 5 | 5 | Latex particles 3 | | | 315 nm | 5.6 |
| Test reagent 6 | 6 | Latex particles 4 | | | 386 nm | 6.2 |
| Test reagent 7 | 7 | Latex particles 5 | | | 230 nm | 6.8 |
| Test reagent 8 | 1 | Same as test reagent 1 | | | | |
| Test reagent 9 | 1 | | | | | |
| Comparative reagent 1 | 8 | Latex particles 1 | 30.0 | - | 339 nm | 26.1 |
| Comparative reagent 2 | 9 | | 0.9 | - | 310 nm | 0.7 |
| Comparative reagent 3 | 10 | Comparative particles 1 | 7.0 | - | 556 nm | 4.9 |
| Comparative reagent 4 | 11 | Carboxy group-containing polystyrene particles | 7.0 | BSA blocking | 432 nm | 118 |

**Table 2-2**

| | Buffer solution | | | | | |
|---|---|---|---|---|---|---|
| | | Nonionic surfactant | Amphoteric surfactant | Amount of NaCl with respect to buffer solution | Amount of NaCl with respect to test reagent *1 | Amount of NaCl with respect to reaction liquid *2 |
| Test reagent 1 | A | Tween 20 | CHAPS | 500 mM | 361 mM | 325 mM |
| Test reagent 2 | | | | | | |
| Test reagent 3 | | | | | | |
| Test reagent 4 | | | | | | |
| Test reagent 5 | | | | | | |
| Test reagent 6 | | | | | | |
| Test reagent 7 | | | | | | |
| Test reagent 8 | B | | | 137 mM | 99 mM | 89 mM |
| Test reagent 9 | C | | - | 500 mM | 361 mM | 325 mM |
| Comparative reagent 1 | A | Same as test reagent 1 | | | | |
| Comparative reagent 2 | | | | | | |
| Comparative reagent 3 | | | | | | |
| Comparative reagent 4 | | | | | | |

| | | | | | | |
|---|---|---|---|---|---|---|
| *1: In the column "Amount of NaCl with respect to test reagent" in Table 2-2, the content of NaCl in a test reagent (the total of a buffer solution and a liquid having dispersed thereinto affinity particles) at the time of the following measurement is shown. *2: In the column "Amount of NaCl with respect to reaction liquid" in Table 2-2, the content of NaCl that is not derived from any human specimen in the following reaction liquid (the total of a serum, a buffer solution, and a liquid having dispersed thereinto affinity particles) is shown. | | | | | | |

### [Evaluation 1: Measurement of Normal Human Serum]

The measurement of normal human serums was performed with the test reagents 1 to 9 and the comparative reagents 1 to 4.

Five kinds of normal human serums A to E (individual serum specimens, purchased from Tennessee Blood Services) and a negative control serum (ACCURUN 810 MULTI-MARKER NEGATIVE CONTROL: Minaris Medical Co., Ltd., hereinafter referred to as "serum N") were used as the normal human serums. Each of the six kinds of normal human serums was subjected to the measurement as described below.

10 Microliters of each of the normal human serums and 65 µL of a buffer solution (any one of the buffer solutions A to C in accordance with a test reagent to be evaluated) were mixed in a cell, and the temperature of the mixture was held at 37°C for 5 minutes. Next, 25 µL of a liquid having dispersed thereinto affinity particles (any one of the liquids 1 to 11 having dispersed thereinto the affinity particles in accordance with the test reagent to be evaluated) was added to the above-mentioned cell, and the contents were mixed and stirred. The absorbances of the mixed liquid (having a volume of 100 µL) immediately after the stirring at the respective wavelengths shown in Tables 3-1 and 3-2 were measured. A spectrophotometer BioSpectrometer manufactured by Eppendorf SE was used in the absorbance measurement. The mixed liquid was left at rest at 37°C for 5 minutes, and then the absorbances thereof at the same wavelengths were measured again, followed by the calculation of the value of an absorbance change amount ΔABS×10,000.

### [Evaluation 2: Measurement of HCV-positive Serum]

The measurement of HCV-positive serums was performed with the test reagents 1 to 9 and the comparative reagents 1 to 4.

### (Sample Preparation)

Sample preparation was performed by using a HCV-positive control serum (ACCURUN Series Infectrol D: Minaris Medical Co., Ltd.) and the negative control serum ("serum N") used in Evaluation 1 as described below.

Liquids obtained by diluting the HCV-positive control serum 2-fold, 4-fold, 8-fold, and 16-fold with the negative control serum ("serum N") were each prepared. The 16-fold diluted liquid is hereinafter referred to as "serum P1," the 8-fold diluted liquid is hereinafter referred to as "serum P2," the 4-fold diluted liquid is hereinafter referred to as "serum P3," the 2-fold diluted liquid is hereinafter referred to as "serum P4," and the HCV-positive control serum is hereinafter referred to as "serum P5." The used HCV-positive control serum is a serum having the following C.O.I value: a reference value measured with Lumipulse Presto Ortho HCV (PrestoII/L2400) is 34.9.

### (Measurement)

Measurement was performed by the same operation as that of Evaluation 1 except that the six kinds of normal human serums used in Evaluation 1 were changed to the serums P1 to P5.

### [Evaluation Result]

The results of Evaluation 1 and Evaluation 2 are shown in Tables 3-1 and 3-2.

In addition, the results of the group of the six kinds of normal human serums in Evaluation 1 and the result of the serum P1 (positive low value) in Evaluation 2 are shown together in each of FIG. 1-1 to FIG. 1-13.

In addition, the results of the serum N (negative control) in Evaluation 1 and of Evaluation 2 (the positive serums P1 to P5) are shown in each of FIG. 2-1 to FIG. 2-3. With regard to the comparative reagent 1, the evaluations of the serums P3 to P5 in Evaluation 2 were not performed because the detection amounts of the serums P1 and P2 in Evaluation 2 were not able to be distinguished from that of the negative serum.

**Table 3-1**

| | Wavelength at which absorbance is measured | Evaluation 1 (measurement of normal human serum) | | | | | |
|---|---|---|---|---|---|---|---|
| | | ΔABS×10,000 | | | | | |
| | | Serum A | Serum B | Serum C | Serum D | Serum E | Serum N (negative control) |
| Test reagent 1 | 572 nm | 40 | 50 | 10 | 40 | 70 | 10 |
| Test reagent 2 | | 20 | 40 | 10 | 0 | 40 | 20 |
| Test reagent 3 | | 80 | 90 | 50 | 100 | 130 | 70 |
| Test reagent 4 | | 40 | 50 | 20 | 40 | 40 | 10 |
| Test reagent 5 | | 20 | 60 | 30 | 30 | 30 | 20 |
| Test reagent 6 | 604 nm | 50 | 90 | 60 | 40 | 70 | 20 |
| Test reagent 7 | 450 nm | 30 | 20 | 10 | 20 | 10 | 20 |
| Test reagent 8 | 572 nm | 120 | 40 | 70 | 150 | 190 | 110 |
| Test reagent 9 | | 50 | 50 | 30 | 40 | 180 | 20 |
| Comparative reagent 1 | 572 nm | 310 | 50 | 160 | 260 | 60 | 40 |
| Comparative reagent 2 | | 40 | 50 | 30 | 10 | 40 | 40 |
| Comparative reagent 3 | 804 nm | 80 | 60 | 50 | 60 | 100 | 40 |
| Comparative reagent 4 | 700 nm | 90 | 130 | 160 | 70 | 100 | 70 |

**Table 3-2**

| | Evaluation 2 (measurement of HCV-positive serum) | | | | |
|---|---|---|---|---|---|
| | ΔABS×10,000 | | | | |
| | Serum P1 | Serum P2 | Serum P3 | Serum P4 | Serum P5 (positive control) |
| | P5 ×1/16 | P5 ×1/8 | P5 ×1/4 | P5 ×1/2 | |
| Test reagent 1 | 270 | 630 | 1,380 | 3,090 | 4,800 |
| Test reagent 2 | 270 | 720 | 1,160 | 1,920 | 2,450 |
| Test reagent 3 | 290 | 820 | 1,860 | 4,290 | 6,820 |
| Test reagent 4 | 320 | 720 | 1,600 | 3,680 | 5,850 |
| Test reagent 5 | 350 | 910 | 1,990 | 4,720 | 7,340 |
| Test reagent 6 | 592 | 1,188 | 2,400 | 4,968 | 7,020 |
| Test reagent 7 | 120 | 260 | 860 | 2,760 | 6,230 |
| Test reagent 8 | 350 | 760 | 1,500 | 3,130 | 4,460 |
| Test reagent 9 | 290 | 590 | 1,180 | 2,750 | 4,280 |
| Comparative reagent 1 | 190 | 230 | not tested | | |
| Comparative reagent 2 | 60 | 190 | 410 | 390 | 330 |
| Comparative reagent 3 | 1,230 | 2,340 | 2,810 | 2,180 | 1,420 |
| Comparative reagent 4 | 620 | 1,350 | 2,740 | 5,310 | 5,230 |

As shown in FIG. 1-1 to FIG. 1-9, the test reagents 1 to 9 of Examples 1 to 9, in each of which the volume-average particle diameter of the affinity particles is 400 nm or less, the amount of the protein carried on the surfaces of the latex particles is from 1.0 µg/mg to 20.0 µg/mg, and the latex particles before the carriage of the antigen each contain a polymer having a structural unit represented by the formula (1), are each excellent in detection sensitivity because the detection amount of the positive low-value serum P1 is larger than the detection amounts of the normal human serum group. In particular, it is found that the test reagents 1 to 7 whose buffer solutions each contain a nonionic surfactant, an amphoteric surfactant, and a sufficient amount of sodium chloride (not derived from any serum, such an amount that the concentration of sodium chloride becomes 325 mM in the reaction liquid, or 361 mM in the test reagent, at the time of the measurement) are each excellent in detection sensitivity because a variation in the normal human serum group is small.

Meanwhile, as shown in FIG. 1-10, the comparative reagent 1 in which the amount of the protein carried on the surfaces of the latex particles was more than 20.0 µg/mg was not able to distinguish the normal human serum group and the positive low-value serum because a variation in the normal human serum group was large, and the detection amount of the positive low-value serum P1 was small. With regard to the comparative reagent 1, the following assumption is made: the antigen on the particles was excessively present to preclude the establishment of a state in which the molecules of the antigen were appropriately distant from each other; and hence a reduction in sensitivity due to the completion of an antigen-antibody reaction in one particle occurred in the positive specimen, and the deterioration of agglutination due to nonspecific adsorption occurred in some normal human specimens.

As shown in FIG. 1-11, in the comparative reagent 2 in which the amount of the protein carried on the particles was less than 1.0 µg/mg, the detection amount of the positive low-value serum P1 was so small that it was difficult to distinguish the serum from the normal human serum group. This is probably due to an insufficient amount of the antigen for detecting target immunoglobulin G.

As shown in FIG. 1-12 and FIG. 1-13, the following results were obtained: as in the test reagents 1 to 9, the comparative reagents 3 and 4 in each of which the volume-average particle diameter of the affinity particles was more than 400 nm were each excellent in detection sensitivity in Evaluation 1.

As shown in FIG. 2-1 and FIG. 2-2, in each of the test reagents 1 to 9 in each of which the volume-average particle diameter of the affinity particles was 400 nm or less, and the amount of the protein carried on the surfaces of the latex particles in the affinity particles was from 1.0 µg/mg to 20.0 µg/mg, an increase in ratio of the HCV-positive serum P5 in the specimen was observed. That is, an increase in detection amount was observed with an increase in amount of the target immunoglobulin G in the specimen.

Meanwhile, as shown in FIG. 2-3, in each of the comparative reagent 2 in which the amount of the protein carried on the surfaces of the latex particles in the affinity particles is less than 1.0 µg/mg, and the comparative reagents 3 and 4 in each of which the volume-average particle diameter of the affinity particles is more than 400 nm, it is found that in a region where the amount of the target immunoglobulin G in the specimen is equal to or more than a certain value, the detection amount thereof does not increase, and hence a sufficient measurement range cannot be secured.

According to the present invention, a test reagent for a latex agglutination method, the reagent being excellent in sensitivity of the detection of target immunoglobulin G in a human specimen and being capable of sufficiently securing a range (measurement range of the amount of immunoglobulin G) in which a detection amount increases with an increase in amount of the target immunoglobulin G can be provided. In addition, a detection method for detecting target immunoglobulin G in a human specimen can be provided.

While the present invention has been described with reference to exemplary embodiments, it is to be understood that the invention is not limited to the disclosed exemplary embodiments.

## Claims

1. An affinity particle for detecting immunoglobulin G in a human specimen by a latex agglutination method,
wherein the affinity particle has a volume-average particle diameter of 400 nm or less,
wherein the affinity particle comprises a latex particle and a protein carried on a surface of the latex particle,
wherein the protein contains an antigen having a molecular weight of 10,000 or more, and
wherein an amount of the protein carried on the surface of the latex particle is 1.0 µg or more and 20.0 µg or less per 1 mg of the affinity particle.

2. The affinity particle according to claim 1, wherein the antigen is one of a HCV antigen or a HBc antigen.

3. The affinity particle according to claim 1 or 2,
wherein the latex particle has a skeleton structure formed of a structural unit derived from a polymerizable monomer,
wherein the skeleton structure includes a structure corresponding to a structure obtained by removing R² from a structural unit represented by the following formula (1), and
wherein the antigen is carried on the surface of the latex particle by being bonded to a group represented by R² in the formula (1): 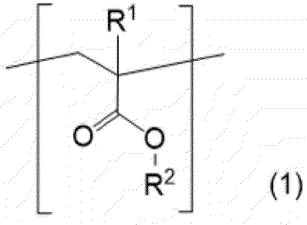 in the formula (1),
R¹ represents a hydrogen atom or a methyl group,
R² represents a group having an epoxy group, a group having a hydroxy group, or a group having a carboxy group, and
R¹ and R² may each vary from structural unit to structural unit.

4. The affinity particle according to claim 3, wherein a content of the structure corresponding to the structure obtained by removing R² from the structural unit represented by the formula (1) in the skeleton structure is 5 mass% or more and less than 50 mass%.

5. The affinity particle according to claim 3 or 4, wherein the structural unit represented by the formula (1) includes a structural unit represented by the following formula (1-A): 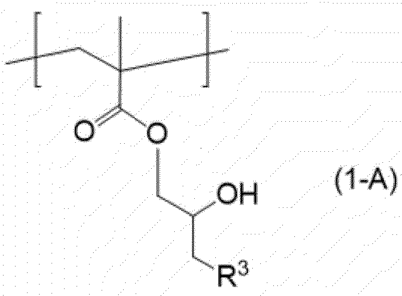 in the formula (1-A), R³ represents a hydroxy group, a group represented by the following formula (1-B), or a group represented by the following formula (1-C): 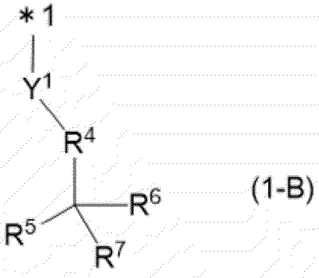 in the formula (1-B),
R⁴ represents a single bond or a methylene group,
R⁵, R⁶, and R⁷ each independently represent a hydrogen atom, a methyl group, a hydroxy group, or a hydroxymethyl group, and the group represented by the formula (1-B) has at least one hydroxy group,
Y¹ represents a sulfur atom or an imino group, and
*1 represents a bonding position; 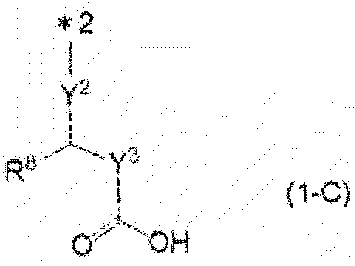 in the formula (1-C),
R⁸ represents a hydrogen atom, a methyl group, a hydroxy group, or a carboxy group,
Y² represents a sulfur atom or an imino group,
Y³ represents a single bond or a methylene group, and
*2 represents a bonding position.

6. The affinity particle according to any one of claims 1 to 5, wherein a ratio of the antigen in the protein carried on the surface of the latex particle is 70 mass% or more.

7. The affinity particle according to any one of claims 1 to 6, wherein the antigen is a chimeric antigen combined with an epitope region.

8. A test reagent comprising a first reagent liquid having dispersed thereinto the affinity particle of any one of claims 1 to 7.

9. The test reagent according to claim 8, further comprising:
a nonionic surfactant and an amphoteric surfactant; and
at least one metal salt selected from sodium chloride, potassium chloride, and magnesium chloride.

10. The test reagent according to claim 9,
wherein the test reagent is a test reagent comprising the first reagent liquid having dispersed thereinto the affinity particle and a second reagent liquid that is a buffer solution, and
wherein the second reagent liquid is free of the affinity particle, contains the nonionic surfactant and the amphoteric surfactant, and further contains the at least one metal salt selected from sodium chloride, potassium chloride, and magnesium chloride.

11. The test reagent according to claim 9 or 10, wherein the nonionic surfactant contains at least one selected from polyoxyethylene sorbitan monolaurate and n-octyl-β-D-glucopyranoside.

12. The test reagent according to any one of claims 9 to 11, wherein the amphoteric surfactant contains at least one selected from 3-[(3-cholamidopropyl)dimethylammonio]-1-propanesulfonate (CHAPS) and 3-[(3-cholamidopropyl)dimethylammonio]-2-hydroxysulfonate (CHAPSO).

13. The test reagent according to any one of claims 10 to 12, wherein at least one of the first reagent liquid and the second reagent liquid contains saccharide.

14. A detection method for detecting immunoglobulin G in a human specimen by a latex agglutination method through use of a test reagent including a first reagent liquid having dispersed thereinto an affinity particle and a second reagent liquid that is a buffer solution, the detection method comprising:
a first step of obtaining a mixed liquid containing the human specimen and the second reagent liquid;
a second step of mixing the mixed liquid and the first reagent liquid to cause the mixed liquid and the first reagent liquid to react with each other, to thereby provide a reaction liquid; and
a third step of measuring a concentration of the immunoglobulin G in the reaction liquid,
wherein the affinity particle has a volume-average particle diameter of 400 nm or less,
wherein the affinity particle includes a latex particle and a protein carried on a surface of the latex particle,
wherein the protein contains an antigen having a molecular weight of 10,000 or more, and
wherein an amount of the protein carried on the surface of the latex particle is 1.0 µg or more and 20.0 µg or less per 1 mg of the affinity particle.

15. The detection method according to claim 14,
wherein the second reagent liquid contains at least one metal salt selected from sodium chloride, potassium chloride, and magnesium chloride, and
wherein a total content of the at least one metal salt selected from sodium chloride, potassium chloride, and magnesium chloride in the second reagent liquid is 120 mM or more and 500 mM or less.

16. The detection method according to claim 14 or 15,
wherein at least one of the first reagent liquid and the second reagent liquid contains at least one metal salt selected from sodium chloride, potassium chloride, and magnesium chloride, and
wherein a total content of the at least one metal salt selected from sodium chloride, potassium chloride, and magnesium chloride with respect to a total of the first reagent liquid and the second reagent liquid is 90 mM or more and 400 mM or less.

17. The detection method according to any one of claims 14 to 16,
wherein the reaction liquid in the third step contains at least one metal salt which is not derived from the human specimen, selected from sodium chloride, potassium chloride, and magnesium chloride, and
wherein a total content of the at least one metal salt which is not derived from the human specimen, selected from sodium chloride, potassium chloride, and magnesium chloride, in the reaction liquid in the third step is 80 mM or more and 360 mM or less.

## Patentansprüche

1. Affinitätspartikel zum Nachweis von Immunglobulin G in einer menschlichen Probe durch ein Latexagglutinationsverfahren,
wobei das Affinitätspartikel einen volumengemittelten Partikeldurchmesser von 400 nm oder weniger aufweist,
wobei das Affinitätspartikel ein Latexpartikel und ein auf einer Oberfläche des Latexpartikels getragenes Protein umfasst,
wobei das Protein ein Antigen mit einem Molekulargewicht von 10.000 oder mehr enthält, und
wobei eine Menge des auf der Oberfläche des Latexpartikels getragenen Proteins 1,0 µg oder mehr und 20,0 µg oder weniger pro 1 mg des Affinitätspartikels beträgt.

2. Affinitätspartikel nach Anspruch 1, wobei das Antigen eines von einem HCV-Antigen oder einem HBc-Antigen ist.

3. Affinitätspartikel nach Anspruch 1 oder 2,
wobei das Latexpartikel eine Skelettstruktur aufweist, die aus einer von einem polymerisierbaren Monomer abgeleiteten Struktureinheit gebildet ist,
wobei die Skelettstruktur eine Struktur enthält, die einer Struktur entspricht, die durch Entfernen von R² aus einer durch die folgende Formel (1) dargestellten Struktureinheit erhalten wird, und
wobei das Antigen auf der Oberfläche des Latexpartikels getragen wird, indem es an eine durch R² in der Formel (1) dargestellte Gruppe gebunden ist: 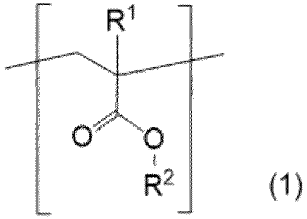 in der Formel (1)
R¹ ein Wasserstoffatom oder eine Methylgruppe darstellt,
R² eine Gruppe mit einer Epoxygruppe, eine Gruppe mit einer Hydroxygruppe oder eine Gruppe mit einer Carboxygruppe darstellt, und
R¹ und R² jeweils von Struktureinheit zu Struktureinheit variieren können.

4. Affinitätspartikel nach Anspruch 3, wobei ein Gehalt der Struktur, welche der Struktur entspricht, die durch Entfernen von R² aus der durch die Formel (1) dargestellten Struktureinheit erhalten wird, in der Skelettstruktur 5 Massen-% oder mehr und weniger als 50 Massen-% beträgt.

5. Affinitätspartikel nach Anspruch 3 oder 4, wobei die durch die Formel (1) dargestellte Struktureinheit eine durch die folgende Formel (1-A) dargestellte Struktureinheit enthält: 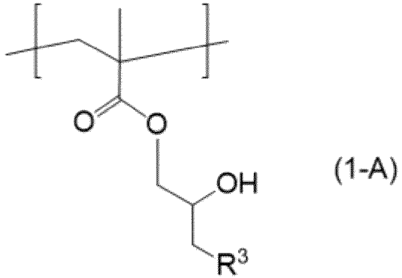 in der Formel (1-A) R³ eine Hydroxygruppe, eine durch die folgende Formel (1-B) dargestellte Gruppe oder eine durch die folgende Formel (1-C) dargestellte Gruppe darstellt: 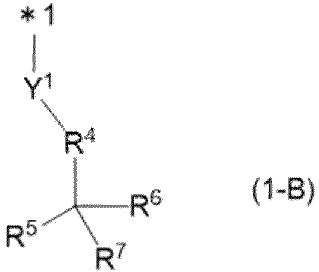 in der Formel (1-B)
R⁴ eine Einfachbindung oder eine Methylengruppe darstellt,
R⁵, R⁶ und R⁷ jeweils unabhängig ein Wasserstoffatom, eine Methylgruppe, eine Hydroxygruppe oder eine Hydroxymethylgruppe darstellen und die durch die Formel (1-B) dargestellte Gruppe mindestens eine Hydroxygruppe aufweist,
Y¹ ein Schwefelatom oder eine Iminogruppe darstellt und
*1 eine Bindungsposition darstellt; 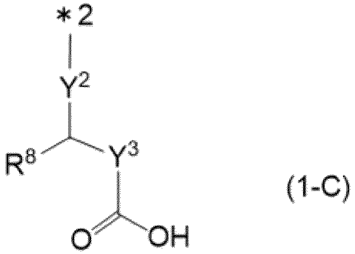 in der Formel (1-C)
R⁸ ein Wasserstoffatom, eine Methylgruppe, eine Hydroxygruppe oder eine Carboxygruppe darstellt,
Y² ein Schwefelatom oder eine Iminogruppe darstellt,
Y³ eine Einfachbindung oder eine Methylengruppe darstellt und
*2 eine Bindungsposition darstellt.

6. Affinitätspartikel nach einem der Ansprüche 1 bis 5, wobei ein Verhältnis des Antigens im auf der Oberfläche des Latexpartikels getragenen Protein 70 Massen-% oder mehr beträgt.

7. Affinitätspartikel nach einem der Ansprüche 1 bis 6, wobei das Antigen ein chimäres Antigen in Kombination mit einer Epitopregion ist.

8. Testreagens, umfassend eine erste Reagensflüssigkeit mit dem darin dispergierten Affinitätspartikel nach einem der Ansprüche 1 bis 7.

9. Testreagens nach Anspruch 8, ferner umfassend:
ein nichtionisches Tensid und ein amphoteres Tensid; und
mindestens ein Metallsalz, ausgewählt aus Natriumchlorid, Kaliumchlorid und Magnesiumchlorid.

10. Testreagens nach Anspruch 9,
wobei das Testreagens ein Testreagens ist, umfassend die erste Reagensflüssigkeit mit dem darin dispergierten Affinitätspartikel und eine zweite Reagensflüssigkeit, die eine Pufferlösung ist, und
wobei die zweite Reagensflüssigkeit frei von dem Affinitätspartikel ist, das nichtionische Tensid und das amphotere Tensid enthält und ferner das mindestens eine Metallsalz, ausgewählt aus Natriumchlorid, Kaliumchlorid und Magnesiumchlorid, enthält.

11. Testreagens nach Anspruch 9 oder 10, wobei das nichtionische Tensid mindestens eines, ausgewählt aus Polyoxyethylensorbitanmonolaurat und n-Octyl-β-D-glucopyranosid, enthält.

12. Testreagens nach einem der Ansprüche 9 bis 11, wobei das amphotere Tensid mindestens eines, ausgewählt aus 3-[(3-Cholamidopropyl)dimethylammonio]-1-propansulfonat (CHAPS) und 3-[(3-Cholamidopropyl)dimethylammonio]-2-hydroxysulfonat (CHAPSO), enthält.

13. Testreagens nach einem der Ansprüche 10 bis 12, wobei mindestens eine der ersten Reagensflüssigkeit und der zweiten Reagensflüssigkeit Saccharid enthält.

14. Nachweisverfahren zum Nachweis von Immunglobulin G in einer menschlichen Probe durch ein Latexagglutinationsverfahren unter Verwendung eines Testreagens, das eine erste Reagensflüssigkeit, in der ein Affinitätspartikel dispergiert ist, und eine zweite Reagensflüssigkeit, die eine Pufferlösung ist, enthält, wobei das Nachweisverfahren umfasst:
einen ersten Schritt des Erhaltens einer gemischten Flüssigkeit, welche die menschliche Probe und die zweite Reagensflüssigkeit enthält;
einen zweiten Schritt des Mischens der gemischten Flüssigkeit und der ersten Reagensflüssigkeit, um zu bewirken, dass die gemischte Flüssigkeit und die erste Reagensflüssigkeit miteinander reagieren, um dadurch eine Reaktionsflüssigkeit bereitzustellen; und
einen dritten Schritt des Messens einer Konzentration des Immunglobulins G in der Reaktionsflüssigkeit,
wobei das Affinitätspartikel einen volumengemittelten Partikeldurchmesser von 400 nm oder weniger aufweist,
wobei das Affinitätspartikel ein Latexpartikel und ein auf einer Oberfläche des Latexpartikels getragenes Protein enthält,
wobei das Protein ein Antigen mit einem Molekulargewicht von 10.000 oder mehr enthält, und
wobei eine Menge des auf der Oberfläche des Latexpartikels getragenen Proteins 1,0 µg oder mehr und 20,0 µg oder weniger pro 1 mg des Affinitätspartikels beträgt.

15. Nachweisverfahren nach Anspruch 14,
wobei die zweite Reagensflüssigkeit mindestens ein Metallsalz, ausgewählt aus Natriumchlorid, Kaliumchlorid und Magnesiumchlorid, enthält, und
wobei ein Gesamtgehalt des mindestens einen Metallsalzes, ausgewählt aus Natriumchlorid, Kaliumchlorid und Magnesiumchlorid, in der zweiten Reagensflüssigkeit 120 mM oder mehr und 500 mM oder weniger beträgt.

16. Nachweisverfahren nach Anspruch 14 oder 15,
wobei mindestens eine der ersten Reagensflüssigkeit und der zweiten Reagensflüssigkeit mindestens ein Metallsalz, ausgewählt aus Natriumchlorid, Kaliumchlorid und Magnesiumchlorid, enthält, und
wobei ein Gesamtgehalt des mindestens einen Metallsalzes, ausgewählt aus Natriumchlorid, Kaliumchlorid und Magnesiumchlorid, in Bezug auf eine Gesamtheit der ersten Reagensflüssigkeit und der zweiten Reagensflüssigkeit 90 mM oder mehr und 400 mM oder weniger beträgt.

17. Nachweisverfahren nach einem der Ansprüche 14 bis 16,
wobei die Reaktionsflüssigkeit im dritten Schritt mindestens ein Metallsalz, welches nicht von der menschlichen Probe abgeleitet ist, ausgewählt aus Natriumchlorid, Kaliumchlorid und Magnesiumchlorid, enthält, und
wobei ein Gesamtgehalt des mindestens einen Metallsalzes, welches nicht von der menschlichen Probe abgeleitet ist, ausgewählt aus Natriumchlorid, Kaliumchlorid und Magnesiumchlorid, in der Reaktionsflüssigkeit im dritten Schritt 80 mM oder mehr und 360 mM oder weniger beträgt.

## Revendications

1. Particule d'affinité pour la détection de l'immunoglobuline G dans un échantillon humain par une méthode d'agglutination au latex,
dans laquelle la particule d'affinité possède un diamètre particulaire moyen en volume de 400 nm ou moins,
dans laquelle la particule d'affinité comprend une particule de latex et une protéine portée sur une surface de la particule de latex,
dans laquelle la protéine contient un antigène ayant un poids moléculaire de 10 000 ou plus, et
dans laquelle une quantité de la protéine portée sur la surface de la particule de latex est de 1,0 µg ou plus et de 20,0 µg ou moins pour 1 mg de la particule d'affinité.

2. Particule d'affinité selon la revendication 1, dans laquelle l'antigène est l'un parmi un antigène VHC ou un antigène HBc.

3. Particule d'affinité selon la revendication 1 ou 2,
dans laquelle la particule de latex possède une structure de squelette formée d'une unité structurelle dérivée d'un monomère polymérisable,
dans laquelle la structure de squelette inclut une structure correspondant à une structure obtenue en retirant R² d'une unité structurelle représentée par la formule (1) suivante, et
dans laquelle l'antigène est porté sur la surface de la particule de latex en étant lié à un groupe représenté par R² dans la formule (1) : 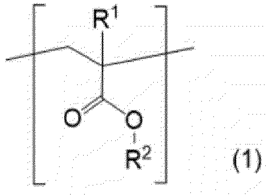 dans la formule (1),
R¹ représente un atome d'hydrogène ou un groupe méthyle ;
R² représente un groupe ayant un groupe époxy, un groupe ayant un groupe hydroxy, ou un groupe ayant un groupe carboxy, et
R¹ et R² peuvent chacun varier d'unité structurelle à unité structurelle.

4. Particule d'affinité selon la revendication 3, dans laquelle une teneur de la structure correspondant à la structure obtenue en retirant R² de l'unité structurelle représentée par la formule (1) dans la structure de squelette est de 5 % en masse ou plus et inférieure à 50 % en masse.

5. Particule d'affinité selon la revendication 3 ou 4, dans laquelle l'unité structurelle représentée par la formule (1) inclut une unité structurelle représentée par la formule (1-A) suivante : 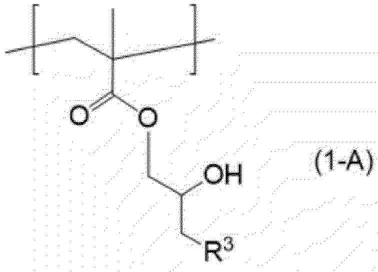 dans la formule (1-A), R³ représente un groupe hydroxy, un groupe représenté par la formule (1-B) suivante, ou un groupe représenté par la formule (1-C) suivante : 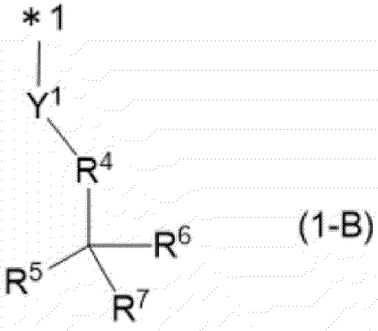 dans la formule (1-B),
R⁴ représente une liaison simple ou un groupe méthylène ; et
R⁵, R⁶, et R⁷ représentent chacun indépendamment un atome d'hydrogène, un groupe méthyle, un groupe hydroxy, ou un groupe hydroxyméthyle, et le groupe représenté par la formule (1-B) possède au moins un groupe hydroxy,
Y¹ représente un atome de soufre ou un groupe imino, et
*1 représente une position de liaison ; 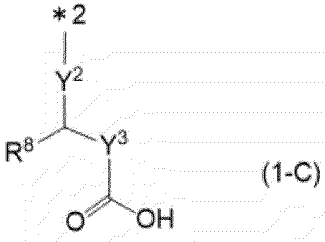 dans la formule (1-C),
R⁸ représente un atome d'hydrogène, un groupe méthyle, un groupe hydroxy ou un groupe carboxy,
^{y2} représente un atome de soufre ou un groupe imino,
Y³ représente une liaison simple ou un groupe méthylène, et
*2 représente une position de liaison.

6. Particule d'affinité selon l'une quelconque des revendications 1 à 5, dans laquelle un rapport de l'antigène dans la protéine portée sur la surface de la particule de latex est de 70 % en masse ou plus.

7. Particule d'affinité selon l'une quelconque des revendications 1 à 6, dans laquelle l'antigène est un antigène chimérique combiné à une région d'épitope.

8. Réactif de test comprenant un premier liquide réactif dans lequel est dispersée la particule d'affinité selon l'une quelconque des revendications 1 à 7.

9. Réactif de test selon la revendication 8, comprenant en outre :
un tensioactif non ionique et un tensioactif amphotère ; et
au moins un sel métallique sélectionné parmi le chlorure de sodium, le chlorure de potassium et le chlorure de magnésium.

10. Réactif de test selon la revendication 9,
dans lequel le réactif de test est un réactif de test comprenant le premier liquide réactif dans lequel est dispersée la particule d'affinité et un deuxième liquide réactif qui est une solution tampon, et
dans lequel le deuxième liquide réactif est exempt de la particule d'affinité, contient le tensioactif non ionique et le tensioactif amphotère, et contient en outre l'au moins un sel métallique sélectionné parmi le chlorure de sodium, le chlorure de potassium et le chlorure de magnésium.

11. Réactif de test selon la revendication 9 ou 10, dans lequel le tensioactif non ionique contient au moins l'un sélectionné parmi le sorbitane monolaurate de polyoxyéthylène et le n-octyl-β-D-glucopyranoside.

12. Réactif de test selon l'une quelconque des revendications 9 à 11, dans lequel le tensioactif amphotère contient au moins l'un sélectionné parmi 3-[(3-cholamidopropyl)diméthylammonio]-1-propanesulfonate (CHAPS) et 3-[(3-cholamidopropyl)diméthylammonio]-2-hydroxysulfonate (CHAPSO).

13. Réactif de test selon l'une quelconque des revendications 10 à 12, dans lequel au moins l'un parmi le premier liquide réactif et le deuxième liquide réactif contient un glucide.

14. Méthode de détection pour la détection de l'immunoglobuline G dans un échantillon humain par une méthode d'agglutination au latex par l'utilisation d'un réactif de test incluant un premier liquide réactif dans lequel est dispersée une particule d'affinité et un deuxième liquide réactif qui est une solution tampon, la méthode de détection comprenant :
une première étape d'obtention d'un liquide mélangé contenant l'échantillon humain et le deuxième liquide réactif ;
une deuxième étape de mélange du liquide mélangé et du premier liquide réactif pour amener le liquide mélangé et le premier liquide réactif à réagir l'un avec l'autre, pour ainsi fournir un liquide de réaction ; et
une troisième étape de mesure d'une concentration de l'immunoglobuline G dans le liquide de réaction,
dans laquelle la particule d'affinité possède un diamètre particulaire moyen en volume de 400 nm ou moins,
dans laquelle la particule d'affinité inclut une particule de latex et une protéine portée sur une surface de la particule de latex,
dans laquelle la protéine contient un antigène ayant un poids moléculaire de 10 000 ou plus, et
dans laquelle une quantité de la protéine portée sur la surface de la particule de latex est de 1,0 µg ou plus et de 20,0 µg ou moins pour 1 mg de la particule d'affinité.

15. Méthode de détection selon la revendication 14,
dans laquelle le deuxième liquide réactif contient au moins un sel métallique sélectionné parmi le chlorure de sodium, le chlorure de potassium et le chlorure de magnésium, et
dans laquelle une teneur totale de l'au moins un sel métallique sélectionné parmi le chlorure de sodium, le chlorure de potassium et le chlorure de magnésium dans le deuxième liquide réactif est de 120 mM ou plus et de 500 mM ou moins.

16. Méthode de détection selon la revendication 14 ou 15, dans laquelle au moins l'un parmi le premier liquide réactif et le deuxième liquide réactif contient au moins un sel métallique sélectionné parmi le chlorure de sodium, le chlorure de potassium et le chlorure de magnésium, et
dans laquelle une teneur totale de l'au moins un sel métallique sélectionné parmi le chlorure de sodium, le chlorure de potassium et le chlorure de magnésium par rapport à un total du premier liquide réactif et du deuxième liquide réactif est de 90 mM ou plus et de 400 mM ou moins.

17. Méthode de détection selon l'une quelconque des revendications 14 à 16,
dans laquelle le liquide de réaction à la troisième étape contient au moins un sel métallique qui n'est pas issu de l'échantillon humain, sélectionné parmi le chlorure de sodium, le chlorure de potassium et le chlorure de magnésium, et
dans laquelle une teneur totale de l'au moins un sel métallique qui n'est pas issu de l'échantillon humain, sélectionné parmi le chlorure de sodium, le chlorure de potassium et le chlorure de magnésium, dans le liquide de réaction à la troisième étape est de 80 mM ou plus et de 360 mM ou moins.
